# EUROPEAN PATENT APPLICATION

(11) **EP 3 427 903 A1**
(43) Date of publication of application: **16.01.2019**
(21) Application number: 18166399.8
(22) Date of filing: 03.06.2013
(51) Int. Cl.: B25J 9/06, B25J 19/06, B25J 13/00

(54) **ARTICULATING SURGICAL INSTRUMENTS**

(30) Priority: 07.06.2012 US 201261656600 P; 09.08.2012 US 201261681340 P; 11.01.2013 US 201361751498 P; 02.05.2013 US 201361818878 P; 20.05.2013 US 201361825297 P
(62) Divisional of application: 13800467.6
(71) Applicant: Medrobotics Corporation, Rayham, Massachusetts 02767 (US)
(72) Inventor: OYOLA, Arnold, Northborough, Massachusetts 01532 (US); CASTRO, Michael Salvatore, Fuquay-Varina, NC 27526 (US); FLAHERTY, J. Christopher, Auburndale, Florida 33823 (US)
(74) Representative: Bittner, Bernhard

(57) **Abstract**

A surgical tool comprises an elongated first assembly and an elongated second assembly. The second assembly comprises an elongated support element, an elongated activation element moveable relative to the support element, and a functional mechanism coupled to the activation element. A movement of the functional mechanism is in response to a movement of the activation element. A force imparted by the movement of the activation element is isolated from the first assembly by the support element.

## Description

### RELATED APPLICATIONS

This application claims the benefit of U.S. Provisional Application No. 61/681,340, filed August 9, 2012, the content of which is incorporated herein by reference in its entirety.

This application claims the benefit of U.S. Provisional Application No. 61/751,498, filed January 11, 2013, the content of which is incorporated herein by reference in its entirety.

This application claims the benefit of U.S. Provisional Application No. 61/656,600, filed June 7, 2012, the content of which is incorporated herein by reference in its entirety.

This application claims the benefit of U.S. Provisional Application No. 61/825,297, filed May 20, 2013, the content of which is incorporated herein by reference in its entirety.

This application claims the benefit of U.S. Provisional Application No. 61818,878, filed May 2, 2013, the content of which is incorporated herein by reference in its entirety.

This application is related to PCT Application No. PCT/US2012/040414, filed June 1, 2012, the content of which is incorporated herein by reference in its entirety.

This application is related to U.S. Provisional Application No. 61/492,578, filed June 2, 2011, the content of which is incorporated herein by reference in its entirety.

This application is related to PCT Application No PCT/US2012/032279, filed April 5, 2012, the content of which is incorporated herein by reference in its entirety.

This application is related to United States Provisional Application No. 61/472,344, filed April 6, 2011, the content of which is incorporated herein by reference in its entirety.

This application is related to PCT Application No PCT/US2011/060214, filed November 10, 2011, the content of which is incorporated herein by reference in its entirety.

This application is related to U.S. Provisional Application No. 61/412,733, filed November 11, 2010, the content of which is incorporated herein by reference in its entirety.

This application is related to PCT Application No PCT/US2012/054802, filed September 12, 2012, the content of which is incorporated herein by reference in its entirety.

This application is related to U.S. Provisional Application No. 61/534,032, filed September 13, 2011, the content of which is incorporated herein by reference in its entirety.

This application is related to U.S. Provisional Application No. 61/406,032, filed October 22, 2010, the content of which is incorporated herein by reference in its entirety.

This application is related to PCT Application No PCT/US2011/057282, filed October 21, 2011, the content of which is incorporated herein by reference in its entirety.

This application is related to U.S. Provisional Application No. 61/368,257, filed July 28, 2010, the content of which is incorporated herein by reference in its entirety.

This application is related to PCT Application No PCT/US2011/044811, filed July 21, 2011, the content of which is incorporated herein by reference in its entirety.

This application is related to PCT Application No PCT/US2012/070924, filed December20, 2012, the content of which is incorporated herein by reference in its entirety.

This application is related to U.S. Provisional Application No. 61/578,582, filed December 21, 2011, the content of which is incorporated herein by reference in its entirety.

This application is related to U.S. Patent Application No. 11/630,279, filed December 20, 2006, published as U.S. Patent Application Publication No. 2009/0171151, the content of which is incorporated herein by reference in its entirety.

### TECHNICAL FIELD

The present inventive concepts generally relate to the field of surgical tools, and more particularly, to articulating surgical tools and tool sheaths, methods of deploying articulating surgical tools and tool sheaths, and methods of forming the same.

### BACKGROUND

As less invasive medical techniques and procedures become more widespread, medical professionals, such as surgeons, may require articulating surgical tools to perform such less invasive medical techniques and procedures from outside the human body. However, conventional articulating surgical tools, such as endoscopes and other types of tools, may have limited turning radii and reduced payload stability at high articulation ranges.

### SUMMARY

Embodiments of the present inventive concepts may be directed to articulating surgical tools and tool sheaths that have extended turning radii and increased payload stability at high articulation ranges.

In one aspect, a system for performing a medical procedure comprises: an articulating probe including inner and outer sleeves; and a surgical tool including a functional element positioned at a distal end of a tool shaft, the tool shaft having an articulation region, wherein the articulating probe and the surgical tool are independently controllable.

In some embodiments, the articulating probe is constructed and arranged to be controlled via a human interface device. The human interface device may include one or more selected from the group consisting of: a haptic controller, a joystick, a track ball, a mouse and an electromechanical device.

In some embodiments, the surgical tool is constructed and arranged to be controlled via a surgical tool handle. The surgical tool handle may include one selected from the group consisting of: scissor handles, a palm-held grip, a thumb/index/middle finger grip and a pistol grip.

In some embodiments, the articulating probe further includes at least one working channel having an opening at a working surface of the articulating probe, the working surface being at a distal end of the articulating probe. A portion of the tool shaft may be positioned within the at least one working channel. The functional element of the surgical tool may extend outwardly from the opening. The functional element may be constructed and arranged to articulate with respect to the working surface of the articulating probe. The functional element may be constructed and arranged to articulate with respect to an axis of extension of the tool shaft. The functional element may be constructed and arranged to articulate between 0° and 90° with respect to the working surface of the articulating probe. The functional element may be constructed and arranged to articulate between 0° and 135° with respect to the working surface of the articulating probe. The functional element may be constructed and arranged to articulate between 0° and 180° with respect to the working surface of the articulating probe.

In some embodiments, the outer sleeve of the articulating probe includes at least one side port. The at least one side port may include a side port lock. The side port lock may include a pneumatic lock. The pneumatic lock may include a solenoid. The pneumatic lock may include an expandable pouch. The side port lock may include a hydraulic lock. The hydraulic lock may include a solenoid. The hydraulic lock may include an expandable pouch or balloon. The side port lock may include an electrically activated lock. The electrically activated lock may include a solenoid. The electrically activated lock may include a piezoelectric actuator. The side port lock may be positioned within the at least one side port. The side port lock may be constructed and arranged to secure a tool shaft that passes through the at least one side port in a locked mode. The side port lock may be constructed and arranged to allow a tool shaft to pass through the at least one side port in an unlocked mode.

In some embodiments, the outer sleeve of the articulating probe includes at least one side port. A portion of the tool shaft may pass through the at least one side port. The side port may guide the tool shaft along an outer surface of the outer sleeve. The functional element of the surgical tool may extend outwardly from a working surface of the articulating probe, the working surface being at a distal end of the articulating probe. The functional element may be constructed and arranged to articulate with respect to the working surface of the articulating probe. The functional element may be constructed and arranged to articulate with respect to an axis of extension of the tool shaft. The functional element may be constructed and arranged to articulate between 0° and 90° with respect to the working surface of the articulating probe. The functional element may be constructed and arranged to articulate between 0° and 135° with respect to the working surface of the articulating probe. The functional element may be constructed and arranged to articulate between 0° and 180° with respect to the working surface of the articulating probe.

In some embodiments, each of the inner and outer sleeves of the articulating probe includes a plurality of probe links.

In some embodiments, the inner sleeve and the outer sleeve of the articulating probe are independently controllable. Each of the inner and outer sleeves of the articulating probe may be configured in one of a limp mode and a rigid mode.

In some embodiments, the articulating probe includes at least one steering cable. The at least one steering cable may terminate at a region proximal to a distal end of the articulating probe.

In some embodiments, the functional element includes one or more selected from the group consisting of: a grasper, a claw, a cutter, a knife, an ablator, a cauterizer, a drug delivery apparatus, a radiation source, an EKG electrode, a pressure sensor, a blood sensor, a camera, a magnet, a heating element and a cryogenic element.

In some embodiments, the functional element includes a first tool sheath cavity and the tool shaft includes a second tool sheath cavity. The surgical tool may be constructed and arranged to provide a cavity path for entry of a second surgical tool. The first tool sheath cavity and second tool sheath cavity may be coupled to form the cavity path. A region of the cavity path may correspond to the articulation region of the tool shaft.

In some embodiments, the surgical tool includes a locking device constructed and arranged to lock an articulated position of the functional element.

In some embodiments, the surgical tool includes a locking device constructed and arranged to lock an operational mode of the functional element.

In some embodiments, the functional element includes a grasper. The grasper may be constructed and arranged to apply a grasping force of approximately 1 lb_{F}. The grasper may be constructed and arranged to apply a grasping force of approximately 1 lb_{F} when the articulation region is positioned in a fully articulated state.

In some embodiments, the system is constructed and arranged to perform a transoral robotic surgery procedure.

In some embodiments, the articulation region of the tool shaft includes at least two segment links. One segment link of the at least two segment links may be unitary. Each segment link of the at least two segment links may be unitary. A first segment link of the at least two segment links may be coupled to a first shaft portion of the tool shaft, and a second segment link of the at least two segment links may be coupled to a second shaft portion of the tool shaft. The functional element may be coupled to the second shaft portion. A first segment link of the at least two segment links may be coupled to a first shaft portion of the tool shaft, and a second segment link of the at least two segment links may be coupled to the functional element. The articulation region of the tool shaft may further include one or more third segment links coupled between the first segment link and the second segment link.

The first segment link may include a body having a first portion and a second portion, wherein the second portion includes a semi-spherical body portion. The first segment link may include a body having a first portion and a second portion, wherein the second portion includes a convex body portion. The convex body portion may be a semi-spherical body portion. The convex body portion may be a semi-ellipsoidal body portion. The first portion may include a cylindrical body portion. The semi-spherical body portion of the first segment link may mate with a semi-spherical cavity portion of the first shaft portion. The semi-spherical body portion of the first segment link may mate with a concave cavity portion of the first shaft portion. The concave cavity portion may be a semi-spherical cavity portion. The concave cavity portion may be a semi-ellipsoidal cavity portion.

The first segment link may include at least one articulation cable channel. The at least one articulation cable channel may include a first opening in an upper surface of the first portion and a second opening in a bottom surface of the first portion. The first portion may include a cylindrical body portion. The at least one articulation cable channel may comprise first through fourth articulation cable channels that may be spaced 90° apart around the circumference or perimeter of the first portion. The at least one articulation cable channel may comprise first through fourth articulation cable channels that may be positioned 90° apart from one another along a common radial path relative to a center axis of the first portion. The first portion may include a cylindrical body portion.

The first segment may include an actuation cable channel. The actuation cable channel may include a first opening at a diametric midpoint of the semi-spherical body portion of the first segment and a second opening at a diametric midpoint of the first portion of the first segment. The first portion may include a cylindrical body portion. The actuation cable channel may include an upper taper joined at the first opening that conforms the first opening with a cylindrical cavity of the body of the first segment. The cylindrical cavity may join a lower taper of the body of the first segment. The lower taper may conform the cylindrical cavity with a semi-spherical cavity of the body of the first segment. The second segment link may include a body having a first portion and a second portion, wherein the second portion includes a semi-spherical body portion.

The second segment link may include a body having a first portion and a second portion, wherein the second portion includes a convex body portion. The convex body portion may be a semi-spherical body portion. The convex body portion may be a semi-ellipsoidal body portion. The first portion may include a cylindrical body portion. The semi-spherical body portion of the second segment link may mate with a semi-spherical cavity portion of the first segment link. The semi-spherical body portion of the second segment link may mate with a concave cavity portion of the first segment link. The concave cavity portion may be a semi-spherical cavity portion. The concave cavity portion may be a semi-ellipsoidal cavity portion. At least two articulation cable channels of the first segment link may be aligned with at least two articulation cable channels of the second segment link. Each articulation cable channel of the first segment link may be aligned with each articulation cable channel of the second segment link.

The body of the second segment link may include at least one articulation cable channel. The at least one articulation cable channel may include a first opening in an upper surface of the first portion and a second opening in a bottom surface of the first portion. The first portion may include a cylindrical body portion. The at least one articulation cable channel may comprise first through fourth articulation cable channels that are spaced 90° apart around the circumference or perimeter of the first portion. The at least one articulation cable channel may comprise first through fourth articulation cable channels that positioned 90° apart from one another along a common radial path relative to a center axis of the first portion. The first portion may include a cylindrical body portion.

The body of the second segment may include an actuation cable channel. The actuation cable channel may include a first opening at a diametric midpoint of the semi-spherical body portion of the second segment and a second opening at a diametric midpoint of the first portion of the second segment. The first portion may include a cylindrical body portion. The actuation cable channel may include an upper taper joined at the first opening that conforms the first opening with a first cylindrical cavity of the body of the second segment. The first cylindrical cavity may join a second cylindrical cavity of the body of the second segment. A diameter of the first cylindrical cavity may be less than a diameter of the second cylindrical cavity.

The second segment link may be coupled to the functional element. The second segment link may be coupled to a connection link of the functional element. The connection link may include a material selected from the group consisting of: metal, plastic, a thermoplastic polymer, stainless steel, polyvinyl chloride and a liquid-crystal polymer.

The functional element may include an actuating piston positioned within an inner cavity of the connection link. The actuation piston may include a material selected from the group consisting of: metal, plastic, a thermoplastic polymer, stainless steel, polyvinyl chloride and a liquid-crystal polymer. The functional element may further include first and second actuation link members coupled to the actuating piston. The first and second actuation link members may include a material selected from the group consisting of: metal, plastic, a thermoplastic polymer, stainless steel, polyvinyl chloride and a liquid-crystal polymer. The functional element may further include first and second claw members respectively coupled to the first and second actuation link members. The first and second claw members may include a material selected from the group consisting of: metal, plastic, a thermoplastic polymer, stainless steel, polyvinyl chloride and a liquid-crystal polymer. Linear movement of the actuating piston within the inner cavity of the connection link may cause the first and second claw members to open and close. An actuating cable may be coupled to the actuating piston. The actuating cable may include one or more selected from the group consisting of: a metal cable, a plastic cable, a sold wire cable, a braided cable and a stainless steel wire braided cable.

The at least two segment links may include a material selected from the group consisting of: metal, plastic, a thermoplastic polymer, stainless steel, polyvinyl chloride, a liquid-crystal polymer and polytetrafluoroethylene. The first segment link may include a material selected from the group consisting of: metal, plastic, a thermoplastic polymer, stainless steel, polyvinyl chloride, a liquid-crystal polymer and polytetrafluoroethylene. The second segment link may include a material different from the first segment link.

In some embodiments, a first segment link of the at least two segment links may be coupled to a first shaft portion of the tool shaft, and a second segment link of the at least two segment links is coupled to one of a second shaft portion of the tool shaft and the functional element. The first shaft portion of the tool shaft includes a cable transitioning segment.

The cable transitioning segment may include at least one articulation cable channel. The at least one articulation cable channel may comprise first through fourth articulation cable channels that are spaced 90° apart around the circumference of the cable transitioning segment. At least two articulation cable channels of the cable transitioning segment may be aligned with at least two articulation cable channels of the first segment link.

The cable transitioning segment may include an actuation cable channel. The actuation cable channel may be positioned at a diametric midpoint of the cable transitioning segment. The cable transitioning segment may include a material selected from the group consisting of: metal, plastic, a thermoplastic polymer, stainless steel, polyvinyl chloride, a liquid-crystal polymer, and polytetrafluoroethylene.

The first shaft portion of the tool shaft may include a flexible tool shaft portion. The flexible tool shaft portion may include a lumen guiding member having at least one cable channel. The at least one cable channel may include an actuating cable channel and at least one articulation cable channel. The actuating cable channel may be positioned at a diametric midpoint of the flexible tool shaft portion, and the at least one articulation cable channel may be positioned along a circumference of the flexible tool shaft portion. The lumen guiding member includes a five lumen stiffening rod. The lumen guiding member may include a material selected from the group consisting of: metal, plastic, a thermoplastic polymer, stainless steel, polyvinyl chloride, a liquid-crystal polymer, and polytetrafluoroethylene.

At least one cavity slot may be formed in the bottom surface of the first portion of the second segment. The at least one cavity slot may include a first cavity slot and a second cavity slot. The first cavity slot may extend from a first articulation cable channel of the at least one articulation cable channel to a second articulation cable channel of the at least one articulation cable channel. A first articulation cable may be positioned within the first articulation cable channel, the first cavity slot and the second articulation cable channel. The first articulation cable may be secured to a surface of the first cavity slot. The first articulation cable may be welded to the surface of the first cavity slot. The first articulation cable may be glued to the surface of the first cavity slot. The first articulation cable may be press fit within the first cavity slot. The second cavity slot may extend from a third articulation cable channel of the at least one articulation cable channel to a fourth articulation cable channel of the at least one articulation cable channel. A second articulation cable may be positioned within the third articulation cable channel, the second cavity slot and the fourth articulation cable channel.

The at least one cavity slot may extend along an entire circumference of the bottom surface of the cylindrical body portion of the second segment. The second opening of the at least one articulation cable channel may be partially defined by the at least one cavity slot. At least one articulation cable may be positioned within the at least one articulation cable channel, and wherein the at least one articulation cable may be secured to a surface of the at least one cavity slot.

In some embodiments, the articulation region of the tool shaft may include a plurality of segment links. Each segment link of the plurality of segment links may be sequentially coupled to another segment link of the plurality of segment links. The plurality of segment links may articulate with respect to one another. A bottom surface of a first portion of a first segment link of the plurality of segment links may abut an upper surface of a first portion of a second segment link of the plurality of segment links to restrict an angle of articulation with respect to a center axis of each of the first and second segment links. The angle of articulation may be restricted to 12° to 15°.

The first portion of the first segment link may include a cylindrical body portion and the first portion of the second segment link may include a cylindrical body portion. Each segment link of the plurality of segment links may be constructed and arranged to provide 12° to 15° of articulation between the functional element and a working surface of the articulating probe. Each segment link of the plurality of segment links may be constructed and arranged to provide 12° to 15° of articulation between the functional element and a longitudinal axis of a cable transitioning segment of the tool shaft. Each segment link of the plurality of segment links may be constructed and arranged to provide 12° to 15° of articulation between the functional element and an axis of extension of the tool shaft.

In some embodiments, the articulation region may be constructed and arranged to support a force of approximately 1 lb_{F} without deflecting more than approximately ½ inch.

In some embodiments, the articulation region may be constructed and arranged to support a force of approximately 1 lb_{F} without deflecting more than approximately ½ inch when in a fully articulated state.

In another aspect, a surgical tool comprises: a functional element positioned at a distal end of a tool shaft; and a tool handle positioned at a proximal end of the tool shaft, wherein the tool shaft includes an articulation region.

In some embodiments, the articulation region may be positioned at the distal end of the tool shaft between the functional element and a first portion of the tool shaft.

In some embodiments, the articulation region may be positioned at a central region of the tool shaft. The articulation region may be positioned between a first portion of the tool shaft and a second portion of the tool shaft. The tool handle may be coupled to a proximal end of the first portion of the tool shaft.

The articulation region may include a plurality of segment links. Each segment link of the plurality of segment links may be constructed and arranged to provide 12° to 15° of articulation between the functional element and an axis of the tool shaft. A first segment link of the plurality of segment links may be coupled to a first portion of the tool shaft and a second segment of the plurality of segment links is coupled to the functional element.

The first segment link may include a body having a first portion and a second portion, wherein the second portion includes a semi-spherical body portion. The first portion may include a cylindrical body portion.

The second segment link may include a body having a first portion and a second portion, wherein the second portion includes a semi-spherical body portion. The first portion may include a cylindrical body portion.

The semi-spherical body portion of the first segment link may mate with a semi-spherical cavity portion of the first portion of the tool shaft and wherein the semi-spherical body portion of the second segment link may mate with a semi-spherical cavity portion of the first segment link. The functional element may include a connection link having a semi-spherical body portion that mates with a semi-spherical cavity portion of the second segment link.

Each of the plurality of segment links may include a body having a first portion and a second portion, wherein the second portion may include a semi-spherical body portion, and wherein each of the plurality of segment links may include at least one articulation cable channel and an actuating cable channel. The first portion may include a cylindrical body portion.

At least one articulation cable may be positioned within the at least one articulation cable channel. The at least one articulation cable may be secured to a distal segment link of the plurality of segment links. A tension applied to the at least one articulation cable may cause the functional element to articulate with respect to the tool shaft.

An actuating cable may be positioned within the actuating cable channel. A tension applied to the actuating cable may cause the functional element to change state.

In some embodiments, the tool shaft includes a five-lumen extrusion positioned within a wire coil. A tool shaft cover may surround the wire coil. The tool shaft cover may include a Pebax®-type shaft cover.

In another aspect, a surgical tool comprises: a functional element positioned at a distal end of a tool shaft; and a tool handle positioned at a proximal end of the tool shaft, wherein the tool shaft includes an articulation region.

In some embodiments, the articulation region may include a plurality of segment links. A first segment link of the plurality of segment links may include a body having first and second concave cavities formed at opposite end surfaces of the body. The first concave cavity may be a semi-spherical cavity. The first concave cavity may be a semi-ellipsoidal cavity. The second concave cavity may be a semi-spherical cavity. The second concave cavity may be a semi-ellipsoidal cavity.

A second segment link of the plurality of segment links may include a body having first and second convex body portions formed at opposite end surfaces of a center body portion. The first convex body portion may be a semi-spherical body portion. The first convex body portion may be a semi-ellipsoidal body portion. The second convex body portion may be a semi-spherical body portion. The second convex body portion may be a semi-ellipsoidal body portion. The center body portion may be cylindrical.

One of the first and second convex body portions of the second segment link may mate with one of the first and second concave cavities of the first segment link. The other of the first and second convex body portions of the second segment link may mate with a concave cavity of the tool shaft. The other of the first and second concave cavities of the first segment link may mate with a convex body portion of a third segment link of the plurality of segment links. The third segment link may be coupled to the functional element.

In some embodiments, a first segment link of the plurality of segment links may include a first body having a first protrusion extending from a surface of the first body. The first body may be a cylindrical body. The first body may have an elliptical cross-section. The first protrusion may be a cylindrical protrusion. The first protrusion may have an elliptical cross-section.

A second segment link of the plurality of segment links may include a second body having a second protrusion extending from a first surface of the second body. The second body may be a cylindrical body. The second body may have an elliptical cross-section. The second protrusion may be a cylindrical protrusion. The second protrusion may have an elliptical cross-section.

The second segment link may include a concave cavity formed in a second surface of the second body. The concave cavity may be a semi-spherical cavity. The concave cavity may be a semi-ellipsoidal cavity. The first protrusion of the first segment link may mate with the concave cavity of the second segment link. The first segment link may be coupled to the functional element. The second protrusion of the second segment link may mate with a concave cavity of the tool shaft.

In some embodiments, a first segment link of the plurality of segment links may include a body having a first body portion and a second body portion. The first body portion may include a cylindrical body portion. The first body portion may have an elliptical cross-section. The second body portion may include a convex body portion. The convex body portion may be a semi-spherical body portion. The convex body portion may be a semi-ellipsoidal body portion.

A second segment link of the plurality of segment links may include a body having center body portion, a convex body portion coupled to a first surface of the center body portion, and a plurality of posts extending outwardly from a second surface of the center body portion. The convex body portion may be a semi-spherical body portion. The convex body portion may be a semi-ellipsoidal body portion. The center body portion may be cylindrical. The center body portion may have an elliptical cross-section. The plurality of posts may be cylindrical. The plurality of posts may have elliptical cross-sections. The plurality of posts may include one of rounded or beveled upper edges.

The plurality of posts may be arranged along a common radial path relative to a center axis the second surface of the center body portion. The plurality of posts may include a center post and two or more outer posts, the center post being positioned at a diametric midpoint of the second surface. The two or more outer posts may be arranged along a common radial path relative to the center post. The outer posts may be equally spaced apart. The two or more outer posts may each have a first height greater than a second height of the center post.

The second body portion of the first segment link may mate with the plurality of posts extending outwardly from the second surface of the center body portion. The convex body portion of the second segment link may mate with a plurality of posts of a third segment link of the plurality of segment links. The third segment link may be coupled to the tool shaft.

In another aspect, a surgical tool comprises an elongated first assembly; and an elongated second assembly comprising: an elongated support element; an elongated activation element moveable relative to the support element; and a functional mechanism coupled to the activation element, a movement of the functional mechanism being in response to a movement of the activation element, wherein a force imparted by the movement of the activation element is isolated from the first assembly by the support element. In some embodiments, the second assembly further comprises a clevis coupled to the support element.

In some embodiments, the clevis is coupled to a distal end of the support element.

In some embodiments, an inner surface of the clevis is coupled to an outer surface of the support element.

In some embodiments, the clevis is bonded to the support element.

In some embodiments, the bond includes an adhesive.

In some embodiments, the clevis is welded to the support element.

In some embodiments, the clevis and the support element are coupled by at least one of swaging, threading, pinning, snap-fitting, press-fitting, or coupling together.

In some embodiments, the activation element moves freely along a direction of extension of the support element and the clevis.

In some embodiments, the surgical tool further comprises a longitudinal clearance between the clevis and a distal end of the first assembly.

In some embodiments, the longitudinal clearance is dimensioned, in a longitudinal direction, to prevent contact between the clevis and the distal end of the first assembly when the force is imparted by the movement of the activation element.

In some embodiments, the dimension of the longitudinal clearance ensures the isolation of the imparted force.

In some embodiments, the dimension of the longitudinal clearance provides for play between the clevis and the distal end of the first assembly when the force is imparted.

In some embodiments, the dimension of the longitudinal clearance is reduced when the force is imparted.

In some embodiments, the surgical tool further comprises a compressible material positioned in the longitudinal clearance.

In some embodiments, the compressible material comprises at least one of elastomer, polymer, rubber, foam, sponge material, or combinations thereof.

In some embodiments, the surgical tool further comprises a compressible element positioned in the longitudinal clearance.

In some embodiments, the compressible element comprises at least one of a spring, a compressible disk, an elastomeric disk, a hydraulic piston, a pneumatic piston, or combinations thereof.

In some embodiments, the clevis includes a base and a protrusion extending from the base.

In some embodiments, the protrusion extends into a recess of a distal end of the first assembly.

In some embodiments, the recess includes an inner end wall and a sidewall.

In some embodiments, a longitudinal clearance is between the protrusion and the inner end wall of the recess.

In some embodiments, the base is wider than the protrusion.

In some embodiments, a longitudinal clearance is between the base of the clevis and the distal end of the first assembly.

In some embodiments, an outer width of the base is equal to an outer width of the distal end of the first assembly.

In some embodiments, the protrusion has a cylindrical outer surface and the distal end has a cylindrical inner surface.

In some embodiments, the cylindrical outer surface of the protrusion includes at least one first flat portion that registers with a corresponding second flat portion of the inner surface of the distal end.

In some embodiments, the registration of the first and second flat portions prevents twisting of the second assembly relative to the first assembly.

In some embodiments, the clevis includes a housing for receiving the functional mechanism.

In some embodiments, the activation element is coupled to the functional mechanism at the housing.

In some embodiments, the housing is dimensioned to permit the functional mechanism to expand and contract relative to the housing during the movement of the functional mechanism.

In some embodiments, the second assembly is in communication with the first assembly so that the support element of the second assembly is movable relative to the first assembly.

In some embodiments, the support element includes a lumen that extends along a direction of extension of the support element.

In some embodiments, the activation element is slidably positioned in the lumen of the support element.

In some embodiments, the activation element slidably communicates with the support element in a direction of extension of the support element.

In some embodiments, the support element is constructed and arranged as a coil.

In some embodiments, the support element is constructed and arranged as a rod.

In some embodiments, the rod has limited compression in a direction of extension of the support element and is flexible in a lateral direction relative to the direction of extension.

In some embodiments, the support element is constructed and arranged as a hollow tube.

In some embodiments, the support element comprises an arrangement of multiple links.

In some embodiments, the support element has limited compression in a direction of extension of the support element and is flexible in a lateral direction relative to the direction of extension.

In some embodiments, the support element absorbs a load caused by the force imparted by the movement of the activation element.

In some embodiments, the support element prevents a force from being applied to the first assembly when the force is imparted by the movement of the activation element.

In some embodiments, the activation element is freely moveable relative to the support element.

In some embodiments, the activation element moves freely within the support element.

In some embodiments, the activation element moves freely proximal to an outer surface of the support element.

In some embodiments, movement of the activation element is induced by a handle at a proximal end of the surgical tool.

In some embodiments, the activation element is constructed and arranged as a wire.

In some embodiments, the wire is constructed and arranged to deliver energy and/or data.

In some embodiments, the activation element is constructed and arranged as a cable.

In some embodiments, the activation element is constructed and arranged as a fiber.

In some embodiments, the fiber is constructed and arranged to deliver energy and/or data.

In some embodiments, the activation element comprises a lubricious outer surface portion.

In some embodiments, the lubricious outer surface portion comprises a material selected from the group consisting of: Teflon®; graphite; a hydrophilic coating; a surface area reducing texture; and combinations thereof.

In some embodiments, the functional mechanism comprises at least one of: a grasper; a scissor; a cutter; a claw; or a knife.

In some embodiments, the functional mechanism comprises at least one of: an ablator, a cauterizer, a drug delivery apparatus, a radiation source, an EKG electrode, a pressure sensor, a blood sensor, a camera, a magnet, a heating element or a cryogenic element. In some embodiments, the functional mechanism comprises a spring-biased tool.

In some embodiments, the spring-biased tool operates to apply a force relative to the movement of the activation element.

In some embodiments, the spring-biased tool operates to apply a force to reset the functional mechanism.

In some embodiments, the spring-biased tool operates to apply a force that is opposite the force imparted by the movement of the activation element.

In some embodiments, the functional element is constructed and arranged to articulate with respect to a direction of extension of the first assembly.

In some embodiments, the functional mechanism includes an actuating piston coupled to the activation element to link the activation element to the functional mechanism.

In some embodiments, the actuating piston is positioned within an inner cavity of a distal and of the first assembly.

In some embodiments, the functional element further includes first and second actuation link members coupled to the actuating piston.

In some embodiments, the first and second actuation link members include a material selected from the group consisting of: metal, plastic, a thermoplastic polymer, stainless steel, polyvinyl chloride; liquid-crystal polymer; and combinations thereof.

In some embodiments, the functional element further includes first and second claw members respectively coupled to the first and second actuation link members. In some embodiments, the first and second claw members include a material selected from the group consisting of: metal, plastic, a thermoplastic polymer, stainless steel, polyvinyl chloride; liquid-crystal polymer; and combinations thereof.

In some embodiments, linear movement of the actuating piston within the inner cavity causes the first and second claw members to open and close.

In some embodiments, the surgical tool further comprises a handle coupled to a proximal end of the support element at a proximal end of the first assembly.

In some embodiments, the handle controls the surgical tool.

In some embodiments, the surgical tool further comprises at least one steering cable that operates to control articulation of the first assembly, In some embodiments, the at least one steering cable is coupled to the handle.

In some embodiments, the surgical tool further comprises a ball and socket mechanism in communication with the handle that controls movement of the at least one steering cable.

In some embodiments, the surgical tool further comprises a locking mechanism that locks a position of the ball and socket mechanism.

In some embodiments, the locking mechanism comprises a threaded nut or a thumb screw.

In some embodiments, the activation element is coupled to the handle.

In some embodiments, the handle includes a trigger and in some embodiments, the activation element is coupled to the trigger.

In some embodiments, the trigger is spring-loaded.

In some embodiments, activation of the trigger moves the activation element in a first direction.

In some embodiments, release of the trigger by an operator causes the trigger to reset in turn allowing the activation element to move in a second direction opposite the first direction.

In some embodiments, the trigger initiates the movement of the activation element in a direction toward the trigger.

In some embodiments, the handle includes a mount at which the support element is coupled.

In some embodiments, the support element is coupled to the mount at a proximal end of the support element.

In some embodiments, the handle further includes a trigger and in some embodiments, the activation element extends through the mount to the trigger.

In some embodiments, the handle includes one selected from the group consisting of: scissor handles; a palm-held grip; a thumb/index/middle finger grip; a pistol grip; a reciprocating trigger; and combinations thereof.

In some embodiments, the surgical tool includes a locking device constructed and arranged to lock an articulated position of the functional mechanism.

In some embodiments, the surgical tool includes a locking device constructed and arranged to lock an operational mode of the functional mechanism.

In some embodiments, the first assembly is adjacent an outer surface of the second assembly.

In some embodiments, the first and second assemblies extend in a same direction of extension.

In some embodiments, the first assembly and the second assembly are co-located in a lumen of an articulating probe.

In some embodiments, at least a portion of the second assembly is positioned in the first assembly.

In some embodiments, the isolation of the force imparted by the movement of the activation element relative to the first assembly by the support element prevents binding at an articulation region of the first assembly.

In some embodiments, the isolation of the force imparted by the movement of the activation element relative to the first assembly by the support element prevents binding of articulation segments at an articulation region of the first assembly.

In some embodiments, the surgical tool is constructed and arranged to be controlled via a human interface device.

In some embodiments, the human interface device includes one selected from the group consisting of: a haptic controller; a joystick; a track ball; a mouse; an electromechanical device; and combinations thereof.

In some embodiments, the first assembly comprises: a tool shaft; an articulation region comprising a plurality of articulation segments; and at least two steering cables extending through the tool shaft to the articulation region.

In some embodiments, the articulation region is at a distal end of the surgical tool.

In some embodiments, the at least two steering cables control an articulation of the articulation segments.

In some embodiments, at least one of the articulation segments includes at least one articulation cable channel through which a steering cable of the at least two steering cables extends.

In some embodiments, the at least one articulation cable channel comprises first through third articulation cable channels that are spaced approximately 20° apart around a circumference or perimeter of the articulation segment.

In some embodiments, the at least one articulation cable channel comprises first through fourth articulation cable channels that are spaced approximately 90° apart around a circumference or perimeter of the articulation segment.

In some embodiments, the at least one articulation cable channel comprises first through fourth articulation cable channels that are spaced approximately 90° apart from one another along a common radial path relative to a center axis of the first assembly.

In some embodiments, the at least two steering cables operate to lock an articulation position of the articulation region.

In some embodiments, the surgical tool comprises a locking mechanism that retains the steering cables in a locked position.

In some embodiments, the force imparted by the movement of the activation element is isolated from the articulation region by the support element.

In some embodiments, the force imparted by the movement of the activation element is independent from an orientation of the articulation region.

In some embodiments, the force imparted by the movement of the activation element is independent from a force applied to the at least two steering cables.

In some embodiments, the isolation of the force imparted by the movement of the activation element from the articulation region prevents binding of the at least two steering cables.

In some embodiments, the isolation of the force imparted by the movement of the activation element from the articulation region prevents inadvertent locking of neighboring articulation segments.

In some embodiments, the isolation of the force imparted by the movement of the activation element from the articulation region avoids movement of the articulation region in response to a movement of the activation element

In some embodiments, the tool shaft includes a lumen guiding member for receiving the activation element and the at least two steering cables.

In some embodiments, the lumen guiding member includes a multi-lumen stiffening rod.

In some embodiments, the multi-lumen stiffening rod includes a first cable channel for receiving the activation element and a plurality of second cable channels for receiving the at least two steering cables.

In some embodiments, the articulation region of the tool shaft includes at least two segment links.

In some embodiments, a first segment link of the at least two segment links is coupled to a first shaft portion of the tool shaft, and a second segment link of the at least two segment links is in communication with the functional mechanism.

In some embodiments, the articulation region of the tool shaft further includes one or more third segment links coupled between the first segment link and the second segment link.

In some embodiments, the first segment link includes a body having a first portion and a second portion, wherein the second portion includes a semi-spherical body portion.

In some embodiments, the first segment link includes a body having a first portion and a second portion, wherein the second portion includes a convex body portion.

In some embodiments, the convex body portion is a semi-spherical body portion.

In some embodiments, the convex body portion is a semi-ellipsoidal body portion.

In some embodiments, the first portion includes a cylindrical body portion.

In some embodiments, a semi-spherical body portion of the first segment link mates with a semi-spherical cavity portion of the first shaft portion.

In some embodiments, a semi-spherical body portion of the first segment link mates with a concave cavity portion of the first shaft portion.

In some embodiments, the second segment link includes a body having a first portion and a second portion, wherein the second portion includes a convex body portion.

In some embodiments, the convex body portion is a semi-spherical body portion.

In some embodiments, the convex body portion is a semi-ellipsoidal body portion.

In some embodiments, the first portion includes a cylindrical body portion.

In some embodiments, a bottom surface of a first portion of a first segment link of the plurality of segment links abuts an upper surface of a first portion of a second segment link of the plurality of segment links to restrict an angle of articulation with respect to a center axis of each of the first and second segment links.

In some embodiments, the angle of articulation is restricted to approximately 12° to 15°.

In some embodiments, each segment link of the at least two is constructed and arranged to provide approximately 12° to 15° of articulation between the functional mechanism and a working surface of the surgical tool.

In some embodiments, each segment link of the at least two is constructed and arranged to provide approximately 12° to 15° of articulation between the functional mechanism and a working surface of the tool shaft.

In some embodiments, the articulation region is constructed and arranged to support a force of 1 lb_{F} without deflecting more than approximately ½ inch.

In some embodiments, the articulation region is constructed and arranged to support a force of approximately 1 lb_{F} without deflecting more than approximately ½ inch when in a fully articulated state.

In some embodiments, a distal end of the support element is positioned at the second segment link.

In some embodiments, the articulation region of the first assembly includes a lumen and wherein a portion of the second assembly is positioned in the lumen of the first assembly.

In some embodiments, each of the at least two steering cables has a proximal end terminating at a surgical tool handle, and wherein the movement of the articulation segments relative to each other is controlled by the surgical tool handle.

In some embodiments, the first assembly is adjacent an outer surface of the second assembly and extends along a common direction of extension as the second assembly.

In some embodiments, the surgical tool further comprises a sheath, the first assembly and the second assembly co-located in a lumen of a sheath.

In some embodiments, the activation element extends along an outer surface of the first assembly.

In another aspect, provided is a method of performing a medical procedure using the surgical tool referred to herein.

In another aspect, provided is a system for performing a medical procedure comprising an articulating probe including inner and outer sleeves; and a surgical tool comprising:
an elongated first assembly; an elongated second assembly comprising:
an elongated support element; an elongated activation element moveable relative to the support element; and a functional mechanism coupled to the activation element, a movement of the functional mechanism being in response to a movement of the activation element,
wherein a force imparted by the movement of the activation element is isolated from the first assembly by the support element, and wherein the articulating probe and the surgical tool are independently controllable.

### BRIEF DESCRIPTION OF THE DRAWINGS

The foregoing and other objects, features and advantages of embodiments of the present inventive concepts will be apparent from the more particular description of preferred embodiments, as illustrated in the accompanying drawings in which like reference characters refer to the same elements throughout the different views. The drawings are not necessarily to scale, emphasis instead being placed upon illustrating the principles of the preferred embodiments.
**FIG. 1A** is a perspective view of an articulating probe of a system for performing a medical procedure, in accordance with embodiments of the present inventive concepts;
**FIGs. 1B and 1C** are end views of a working surface of the articulating probe illustrated at FIG. 1A, in accordance with embodiments of the present inventive concepts;
**FIG. 2** is a perspective view of an articulating surgical tool, in accordance with embodiments of the present inventive concepts;
**FIGs. 3A** **and** **3B** are perspective views of a distal end of the articulating surgical tool illustrated at FIG. 2, in accordance with embodiments of the present inventive concepts;
**FIG. 4A** is a perspective view of segment links of the articulating surgical tool illustrated at FIG. 3, in accordance with embodiments of the present inventive concepts;
**FIG. 4B** is a perspective view of segment links of the articulating surgical tool illustrated at FIG. 3, in accordance with embodiments of the present inventive concepts;
**FIG. 4C** is a cross-sectional perspective view of the segment links illustrated at FIG. 4, in accordance with embodiments of the present inventive concepts;
**FIGs. 5A** **and** **5B** are perspective views illustrating articulation ranges of the articulating surgical tool illustrated at FIG. 2, in accordance with embodiments of the present inventive concepts;
**FIG. 6A** is a side perspective view illustrating an alternative segment link configuration of an articulating surgical tool, in accordance with embodiments of the present inventive concepts;
**FIG. 6B** is a side perspective view illustrating an alternative segment link configuration of an articulating surgical tool, in accordance with embodiments of the present inventive concepts;
**FIG. 6C** is a sectional view of the third segment links illustrated in FIG. 6B, in accordance with embodiments of the present inventive concepts;
**FIG. 7** is a perspective view illustrating an alternative segment link configuration of an articulating surgical tool, in accordance with embodiments of the present inventive concepts;
**FIG. 8A** is a perspective view illustrating an alternative segment link configuration of an articulating surgical tool, in accordance with embodiments of the present inventive concepts;
**FIG. 8B** is a perspective view of a segment link, in accordance with embodiments of the present inventive concepts;
**FIG. 8C** is a top view of the segment link illustrated in FIG. 8B, in accordance with embodiments of the present inventive concepts;
**FIG. 8D** is a perspective view of a segment link, in accordance with embodiments of the present inventive concepts;
**FIG. 8E** is a top view of the segment link illustrated in FIG. 8D, in accordance with embodiments of the present inventive concepts;
**FIG. 9** is a perspective view illustrating an alternative segment link configuration of an articulating surgical tool, in accordance with embodiments of the present inventive concepts;
**FIG. 10** is a cross-sectional side view of an articulating surgical tool, in accordance with another embodiment of the present inventive concepts;
**FIG. 11** is a detailed view of the articulating surgical tool illustrated in FIG. 10, in accordance with another embodiment of the present inventive concepts;
**FIG. 12** is an oblique view of the articulating surgical tool of FIGs. 10 and 11, in accordance with another embodiment of the present inventive concepts;
**FIG. 13** is an expanded view of the handle assembly illustrated in FIG. 10, in accordance with another embodiment of the present inventive concepts;
**FIG. 14A** is a cross-sectional side view of an articulating surgical tool having a functional element in a first state, in accordance with an embodiment of the present inventive concepts;
**FIG. 14B** is a cross-sectional side view of the articulating surgical tool of FIG. 14A, where the functional element is in a second state; and
**FIGs. 15A-15C** are cross-sectional side views of an articulating surgical tool, each side view representing a different state of the articulating surgical tool, in accordance with another embodiment of the present inventive concepts.

### DETAILED DESCRIPTION OF EMBODIMENTS

The terminology used herein is for the purpose of describing particular embodiments and is not intended to be limiting of the inventive concepts. As used herein, the singular forms "a," "an" and "the" are intended to include the plural forms as well, unless the context clearly indicates otherwise. It will be further understood that the terms "comprises," "comprising," "includes" and/or "including," when used herein, specify the presence of stated features, integers, steps, operations, elements, and/or components, but do not preclude the presence or addition of one or more other features, integers, steps, operations, elements, components, and/or groups thereof.

It will be understood that, although the terms first, second, third etc. may be used herein to describe various limitations, elements, components, regions, layers and/or sections, these limitations, elements, components, regions, layers and/or sections should not be limited by these terms. These terms are only used to distinguish one limitation, element, component, region, layer or section from another limitation, element, component, region, layer or section. Thus, a first limitation, element, component, region, layer or section discussed below could be termed a second limitation, element, component, region, layer or section without departing from the teachings of the present application.

It will be further understood that when an element is referred to as being "on" or "connected" or "coupled" to another element, it can be directly on or above, or connected or coupled to, the other element or intervening elements can be present. In contrast, when an element is referred to as being "directly on" or "directly connected" or "directly coupled" to another element, there are no intervening elements present. Other words used to describe the relationship between elements should be interpreted in a like fashion (e.g., "between" versus "directly between," "adjacent" versus "directly adjacent," etc.). When an element is referred to herein as being "over" another element, it can be over or under the other element, and either directly coupled to the other element, or intervening elements may be present, or the elements may be spaced apart by a void or gap.

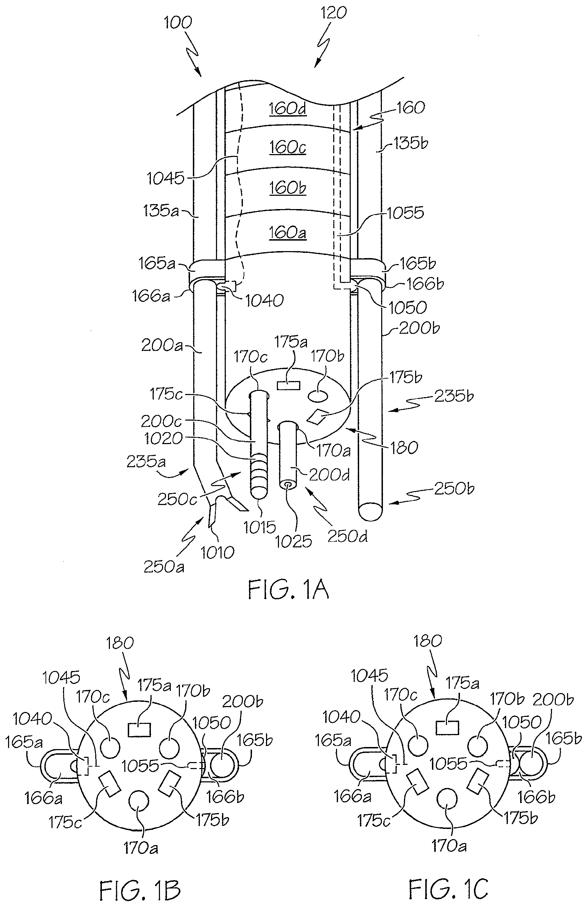

**FIG. 1A** is a perspective view of an articulating probe of a system for performing a medical procedure, and **FIGs. 1B and 1C** are end views of a working surface of the articulating probe illustrated at FIG. 1A. A system 100 for performing a medical procedure, such as a transoral robotic surgery procedure, may include an articulating probe 120 for guiding one or more surgical tools 200, 200a-d and/or tool sheaths 200, 200a within a patient body. The system 100 may include one or more features of the surgical positioning and support system described in U.S. Provisional Patent Application Serial No. 61/368,257, filed July 28, 2010 corresponding to PCT application serial number PCT/US2011/044811, filed July 21, 2011, the contents of which are herein incorporated by reference in their entirety.

An operator, such as a medical professional, may control the articulating probe 120 via a human interface device (HID) to manipulate or otherwise control the functions and movement of the articulating probe 120. The HID may include one selected from the group consisting of: a haptic controller, a joystick, a track ball, a mouse and an electromechanical device.

The articulating probe 120 may include an inner sleeve (not shown) and an outer sleeve 160, which can advance or retract with respect to one another during manipulation of the articulating probe 120. For example, the inner and outer sleeves of the articulating probe 120, which may include a plurality of inner links and a plurality of outer links 160, 160a-d, can be configured in one of a limp mode and a rigid mode so as to facilitate the manipulation of the articulating probe 120. For example, the inner and outer sleeves may be configured in one of the limp mode and the rigid mode via one or more steering cables of the articulation probe 120.

Exemplary probes are further described in U.S. Patent Application Publication No. 2009/0171151, published on July 2, 2009, by Choset, et al., and U.S. Patent Application Publication No. 2008/0039690, published February 14, 2008, by Zubiate, et al., the contents of each being herein incorporated by reference in their entirety.

The articulating probe 120 may include at least one working channel 170, 170a-c having an opening at a working surface 180 of the articulating probe 120. The working channel 170, 170a-c may extend throughout the articulating probe 120, for example, from a proximal end to a distal end of the articulating probe 120. The working surface 180 may be positioned at a distal end of the articulating probe 120. For example, the working surface 180 may be positioned at a distal end of an outer distal link 160a of the articulating probe 120.

The articulating probe 120 may include at least one side port or guide hole 166, 166a-b. For example, in the embodiments shown at FIG. 1, the articulating probe 120 includes first and second side ports 166a, 166b formed in flanges 165a, 165b of an outer link 160a. The articulating probe 120 may further include at least one feed tube 135, 135a-b coupled to the side port or guide hole 166, 166a-b of the articulating probe 120.

Although first and second side ports 166a, 166b are shown at FIG. 1A, a plurality of first and second side ports 166a, 166b may be formed in a plurality of flanges 165a, 165b of the articulating probe 120. For example, multiple first and/or second side ports 166a, 166b may be positioned along the outer sleeve 160 of the articulating probe 120 so as to provide a guide for one or more feed tubes 135, 135a-b that articulate in common with the articulating probe 120.

The articulating probe may include one or more light sources 175, 175a-c provided at the working surface 180 of the articulating probe 120. The light sources 175, 175a-c may include electron stimulated light sources such as electron stimulated luminescence light sources, incandescent light sources such as incandescent light bulbs, electroluminescent light sources such as light-emitting diodes, and gas discharge light sources such as fluorescent lamps.

The light sources 175, 175a-c may further include optical fibers, which can be configured to transmit light to and from the working surface 180 of the articulating probe 120.

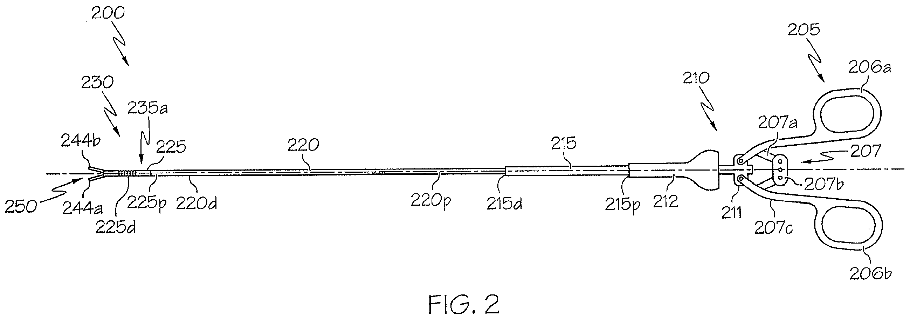

The system 100 may further include one or more surgical tools 200, 200a-d having an articulation region 235, 235a-b. The system 100 may be configured to allow an operator to independently control the articulating probe 120 and the surgical tools 200, 200a-d. For example, the articulating probe 120 may be controlled via a HID and the surgical tools 200, 200a-d may be controlled via a tool handle (see for example, tool handle 205 shown at FIG. 2).

The system 100 may be configured with any number of surgical tools 200, 200a-d, which can be slidably positioned within a working channel 170, 170a-c of the articulating probe 120 and/or a side port 166, 166a-b or guide hole 166, 166a-b of the articulating probe 120.

The articulating probe 120 may be configured to guide one or more surgical tools 200, 200a-d, for example, during a medical procedure. For example, prior, during or after a medical procedure, a portion of the surgical tool shaft may be positioned within at least one of the working channels 170, 170a-c of the articulating probe 120. The articulating probe 120 may be further configured to allow an operator to slidably position the surgical tool shaft within at least one of the working channels 170, 170a-c so that a functional element 250, 250a-b of the surgical tool 200, 200a-d can be extended outwardly from a working channel opening.

In a further example, prior, during or after a medical procedure, a portion of the surgical tool shaft may be positioned within at least one side port or guide hole 166, 166a-b of the articulating probe 120. The articulating probe 120 may be further configured to allow an operator to slidably position the surgical tool shaft within at least one of the side ports or guide holes 166, 166a-b so that a functional element 250, 250a-b of the surgical tool 200, 200a-d can be extended outwardly from the working surface 180 of the articulating probe 120. A portion of the surgical tool shaft may pass through at least one side port or guide hole 166, 166a-b of the articulating probe 120, such that the side port or guide hole 166, 166a-b guides the surgical tool shaft along an outer surface of the outer sleeve 160 of the articulating probe 120.

The articulating probe 120 may include side port or guide hole locks 1040, 1050, which can be configured in one of a locked or unlocked mode. The lock 1040, 1050 may be constructed to secure a position of a surgical tool 200, 200a-b positioned within the side ports or guide holes 166, 166a-b of the articulating probe 120, thus preventing the surgical tool 200, 200a-b from sliding within the side ports or guide holes 166, 166a-b.

In some embodiments, the articulating probe 120 may include a pneumatic or hydraulic lock 1050, such as a solenoid or air/fluid pouch. For example, the pneumatic or hydraulic lock 1050 may be positioned within the side ports or guide holes 166, 166a-b of the articulating probe 120. The articulating probe 120 may further comprise a channel or tube 1055 for supplying pressurized gas or liquid to the pneumatic or hydraulic lock 1050.

In some embodiments, the articulating probe 120 may include an electrically activated lock 1040, such as a solenoid, piezoelectric actuator or nitinol actuated lock. For example, the electrically activated lock 1040 may be positioned within the side ports or guide holes 166, 166a-b of the articulating probe 120. The articulating probe 120 may further comprise a conductor 1045 such as a wire or cable for supplying an actuating signal to the electrically activated lock 1040.

Referring to FIG. 1B, the electrically activated lock 1040 is shown positioned within the first side port or guide hole 166a of the articulating probe 120, and the pneumatic or hydraulic lock 1050 is shown positioned within the second side port or guide hole 166b of the articulating probe 120. In this illustration, the electrically activated lock 1040 and the pneumatic or hydraulic lock 1050 are shown in the unlocked mode so as to allow an operator or user of the system 100 to slidably position a surgical tool shaft within the side port or guide holes 166, 166a-b of the articulating probe 120.

Referring to FIG. 1C, the pneumatic or hydraulic lock 1050 is shown in the locked mode. In the locked mode, the pneumatic or hydraulic lock 1050 expands within the side port or guide hole 166, 166b so as to secure the shaft of the surgical tool 200, 200b within the side port or guide hole 166, 166b. Although not shown, the electrically activated lock 1040 may be configured to expand within the side port or guide hole 166, 166a so as to secure the shaft of the surgical tool 200, 200a within the side port or guide hole 166, 166a.

The functional element 250, 250a-b may be constructed and arranged to articulate with respect to the working surface 180 of the articulating probe 120. For example, in the embodiments shown at FIG. 1A, the functional elements 250a, 250b are shown articulated with respect to the working surface 180 of the articulating probe 120. The functional elements 250, 250a, 250b may also be constructed and arranged to articulate with respect to an axis of extension of the tool shaft.

The functional element 250, 250a-b may be constructed and arranged to articulate between 0° and 90° with respect to the working surface 180 of the articulating probe 120 and/or an axis of extension of the tool shaft. The functional element 250, 250a-b may be constructed and arranged to articulate between 0° and 135° with respect to the working surface 180 of the articulating probe 120 and/or an axis of extension of the tool shaft. The functional element 250, 250a-b may be constructed and arranged to articulate between 0° and 180° with respect to the working surface 180 of the articulating probe 120 and/or an axis of extension of the tool shaft. The functional element 250, 250a-b may be constructed and arranged to articulate at an angle greater than 180° with respect to the working surface 180 of the articulating probe 120 and/or an axis of extension of the tool shaft.

The functional element 250 may include one or more selected from the group consisting of: a grasper, a claw, a cutter, a knife, an ablator, a cauterizer, a drug delivery apparatus, a radiation source, an EKG electrode, a pressure sensor, a blood sensor, a camera, a magnet, a heating element and a cryogenic element. For example, the functional element 250a of a first surgical tool 200a may include a cutter having fist and second blades 1010. The functional element 250b of a second surgical tool 200b may include a heating element, cryogenic element, a pressure sensor, a blood sensor and/or a radiation source 1030. The functional element 250c of a third surgical tool 200c may include one or more EKG electrodes or heart defibrillator electrodes 1015, 1020. The functional element 250d or a fourth surgical tool 200d may include a camera 1025.

**FIG. 2** is a perspective view of an articulating surgical tool. A surgical tool 200 may include a tool handle 205, a surgical tool shaft 215, 220 having an articulation region 235 and a functional element 250.

The surgical tool 200 may be constructed and arranged to be controlled via a surgical tool handle 205. The surgical tool handle 205 may include one selected from the group consisting of: scissor handles, a palm-held grip, a thumb/index/middle finger grip and a pistol grip. For example, in the embodiment shown in FIG. 2, the surgical tool handle 205 includes first and second actuating handle elements 206a, 206b that are coupled at a ball mechanism 211 and a handle link mechanism 207. The handle link mechanism 207 may include first and second links 207a, 207c that are coupled at a link body 207b.

In this exemplary embodiment, the surgical tool includes a ball mechanism 211 that is constructed and arranged to be coupled to a socket mechanism 212 for manipulating or otherwise controlling the functions and movement of the surgical tool 200. Although not shown, one or more articulation cables 410 may be secured to the ball mechanism 211, and one or more actuating cables 420 may be secured to the link body 207b of the handle link mechanism 207. In this manner, a movement of the ball mechanism 211 with respect to the socket mechanism 212 can provide tension or slack on one or more of the articulation cables 410 secured to the ball mechanism 211, thereby adjusting an articulation state of articulation region 235. In addition, a scissoring movement of the first and second actuating handle elements 206a, 206b can cause the link body 207b of the link mechanism 207 to extend outwardly (e.g., along the longitudinal axis) from the ball mechanism 211, thereby applying a tension on the one or more actuating cables 420.

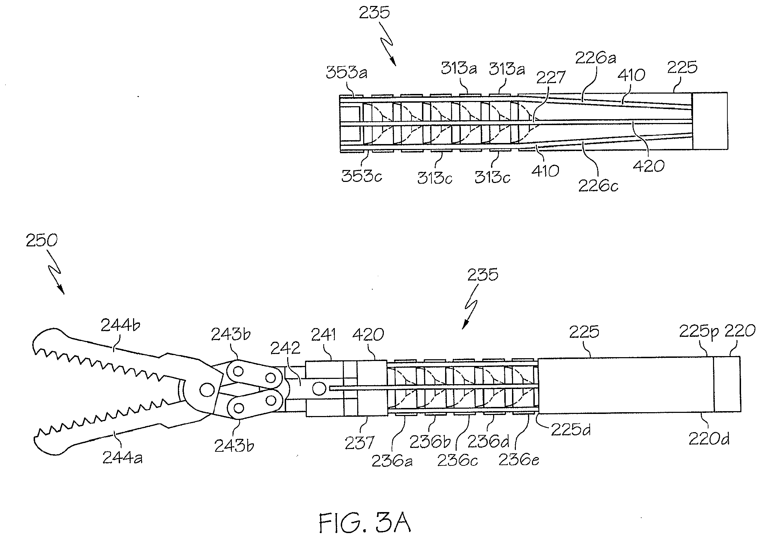

The surgical tool shaft 215, 220 may include a first tool shaft 215 and second tool shaft 220. A proximal end 215p of the first tool shaft 215 may be coupled to the tool handle 205, for example, via the ball and socket mechanisms 211, 212, and a distal end 215d of the first tool shaft 215 may be coupled to a proximal end 220p of the second tool shaft 220. A distal end 220d of the second tool shaft 220 may be directly coupled to the articulation region 235 or indirectly coupled to the articulation region 235. For example, in the embodiments shown in FIGs. 2 and 3A, the distal end 220d of the second tool shaft 220 is coupled to the articulation region 235 through an optional cable transitioning segment 225.

Although the articulation region 235 is shown at a distal end 230 of the surgical tool 200, the articulation region 235 may be provided at any position between the functional element 250 and the proximal end 215p of the first tool shaft 215.

The articulation region 235 may be constructed and arranged to support a force of approximately 1 lb_{F} without deflecting more than approximately ½ inch. In some embodiments, the articulation region 235 is constructed and arranged to support a force of approximately 1 lb_{F} without deflecting more than approximately ½ inch when in a fully articulated state.

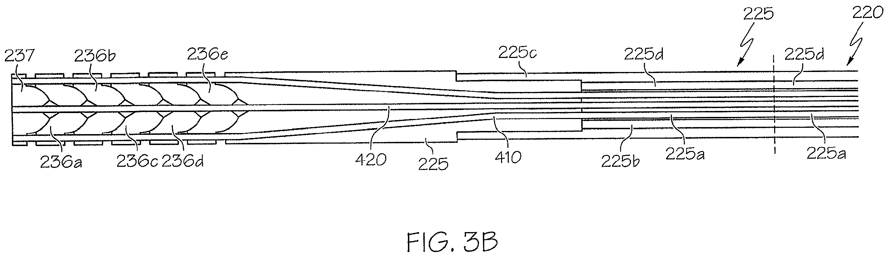

Referring to FIGs. 3A and 3B, the cable transitioning segment 225 may include at least one articulation cable channel 226. For example, the at least one articulation cable channel 226 may include first through fourth articulation cable channels 226 that are spaced approximately 90° apart around the circumference or perimeter of the cable transitioning segment 225. Alternatively, the at least one articulation cable channel 226 may include first through third articulation cable channels 226 that are spaced approximately 120° apart around the circumference or perimeter of the cable transitioning segment 225. At least two articulation cable channels 226a, 226c of the cable transitioning segment 225 may be aligned with at least two articulation cable channels 313a, 313c of a first segment link 236 of the articulation region 235 and/or at least two articulation cable channels 353a, 353c of a second segment link 237 of the articulation region 235. In this manner, one or more articulation cables 410 can be positioned within the cable channels 313a, 313c, 353a, 353c of the first and second segment links 236, 237.

In some embodiments, the cable transitioning segment 225 may include n number of articulation cable channels 226, where n is a real number greater than 0. For cases where n is greater than 1, the n number of articulation cable channels 226 may be evenly spaced apart around the circumference or perimeter of the cable transitioning segment 225 or it may not.

The cable transitioning segment 225 may include an actuation cable channel 227. The actuation cable channel 227 may be positioned at a diametric midpoint of the cable transitioning segment 225, and may be aligned with one or more actuation cable channels 314, 354 of one or more segment links 236, 237 of the articulation region 235. In this manner, one or more actuation cables 420 can be positioned within the cable channels 314, 354 of the first and second segment links 236, 237.

The cable transitioning segment 225 may include a material selected from the group consisting of: metal, plastic, a thermoplastic polymer, stainless steel, polyvinyl chloride, a liquid-crystal polymer, polytetrafluoroethylene, and a combination of these materials or other suitable material.

Referring to FIG. 3B, the cable transitioning segment 225 and/or the surgical tool shaft 220 may comprise a lumen extrusion 225a positioned within a wire coil 225b, such as a flat wire coil or spring. The wire coil 225b may increase a stiffness of the cable transitioning segment 225 and/or the surgical tool shaft 220 so as to prevent twisting and/or kinking of the surgical tool 200. The wire coil 225b may further increase a radial stiffness of the cable transitioning segment 225 and/or the surgical tool shaft 220 so as to prevent a radial collapse of tool shaft and/or to prevent pinching the cables 410, 420. A tool shaft cover 225c such as a Pebax®-type shaft cover may be provided to cover the wire coil 225b.

Referring back to FIG. 2, the first tool shaft 215 may include a rigid tool shaft and the second tool shaft 220 may include a flexible tool shaft; however, the tool shafts 215, 220 of the surgical tool 200 may both include rigid or flexible tool shafts. That is, the first and second tool shafts 215, 220 of the surgical tool 200 may include any combination of rigid and flexible tool shafts.

The flexible tool shafts 215, 220 may include a lumen guiding member having at least one cable channel. In some embodiments, the at least one cable channel includes an actuating cable channel and at least one articulation cable channel. The actuating cable channel may be positioned at a diametric midpoint of the flexible tool shaft, and the at least one articulation cable channel may be positioned along a circumference or perimeter of the flexible tool shaft portion. For example, the lumen guiding member may include a five lumen stiffening rod having an actuating cable channel and first through fourth articulation cable channels.

The lumen guiding member may include a material selected from the group consisting of: metal, plastic, a thermoplastic polymer, stainless steel, polyvinyl chloride, a liquid-crystal polymer, polytetrafluoroethylene, and a combination of these materials or other suitable material.

The functional element 250 of the surgical tool 200 may be provided at the distal end 230 of the surgical tool 200. The functional element 250 may include one or more selected from the group consisting of: a grasper, a claw, a cutter, a knife, an ablator, a cauterizer, a drug delivery apparatus, a radiation source, an EKG electrode, a pressure sensor, a blood sensor, a camera, a magnet, a heating element and a cryogenic element. For example, in the embodiments shown at FIGs. 2 and 3, the functional element 250 includes a grasper having first and second grasping members 244a, 244b. The grasper may be constructed and arranged to apply a grasping force of approximately 1 lb_{F}. The grasper may be further constructed and arranged to apply a grasping force of approximately 1 lb_{F} when the articulation region is positioned in a fully articulated state. The grasper may be further constructed and arranged to apply a substantially similar grasping force throughout all articulation states of the articulation region 235 of the surgical tool 200 so that the operation of the grasper, or other type of functional element 250, is substantially maintained throughout the range of articulation of the surgical tool 200.

The surgical tool 200 may include a locking device that is constructed and arranged to lock an articulated position of the functional element 250. The surgical tool may further include a locking device that is constructed and arranged to lock an operational mode of the functional element 250. For example, the locking device can be constructed and arranged to lock the articulation state of the surgical tool 200, 200a-b and/or grasping state of the functional element 250 (e.g., opened, closed, partially closed).

The surgical tool 200 may be constructed and arranged to provide a cavity path for entry of a second surgical tool, such as a laser fiber or other elongate tool. For example, the functional element of a first surgical tool may include a first tool sheath cavity and the tool shaft of the first surgical tool may include a second tool sheath cavity. In this manner, a second surgical tool may be slidably positioned within the cavity path of the first surgical tool.

For example, referring to FIG. 1A, the first surgical tool 200a may be configured as a surgical tool sheath. The surgical tool sheath may have a sheath opening 165a formed at a distal end of the surgical tool 200a. A second surgical tool may be slidably positioned within a cavity path of the first surgical 200a so that a functional element of the second surgical can extend outward from the sheath opening 165a.

**FIGs. 3A** **and** **3B** are perspective views of a distal end of the articulating surgical tool illustrated at FIG. 2. The articulation region 235 of the tool shaft may include a single segment link 236 or 237 or at least two segment links 236, 237. For example, in the embodiments shown at FIGs. 2, 3A and 3B, the articulation region 235 includes first through sixth segment links 236a-e, 237. The segment links 236, 236a-e, 237 may each be unitary in form, or may each be constructed of multiple portions of material that are bonded or coupled together.

A first segment link 236, 236e of the at least two segment links may be coupled directly or indirectly to the tool shaft 215, 220. For example, the first segment link 236, 236e of the at least two segment links 236, 237 may be coupled to the second tool shaft 220 via the cable transitioning segment 225, which may distribute multiple cables (e.g., one or more actuating cables 420 and/or one or more articulation cables 410) from the tool shaft 215, 220 to the channels of the segment links 236, 237.

A second segment link 237 of the at least two segment links 236, 237 may be coupled to the functional element 250. However, as described above, the articulation region 235 may be provided at any position between the functional element 250 and the proximal end 215p of the first tool shaft 215. For example, the first segment link 236, 236e of the at least two segment links 236, 237 may be coupled directly or indirectly to the first tool shaft 215, and a second segment link 237 of the at least two segment links may be coupled directly or indirectly to the second tool shaft 220.

The second segment link 237 may be coupled to the functional element 250. For example, the second segment link 237 may be coupled to a connection link 241 of the functional element 250. The connection link 241 may include a material selected from the group consisting of: metal, plastic, a thermoplastic polymer, stainless steel, polyvinyl chloride and a liquid-crystal polymer.

The functional element 250 may include an actuating piston 242 positioned within an inner cavity of the connection link 241. The actuation piston 242 may include a material selected from the group consisting of: metal, plastic, a thermoplastic polymer, stainless steel, polyvinyl chloride and a liquid-crystal polymer.

The functional element 250 may further include first and second actuation link members 243a-b coupled to the actuating piston 242. The first and second actuation link members may include a material selected from the group consisting of: metal, plastic, a thermoplastic polymer, stainless steel, polyvinyl chloride and a liquid-crystal polymer.

The functional element 250 may further include first and second claw members or grasper members 244a-b, which can be respectively coupled to the first and second actuation link members 243a-b. The first and second claw members or grasper members 244a-b may include a material selected from the group consisting of: metal, plastic, a thermoplastic polymer, stainless steel, polyvinyl chloride and a liquid-crystal polymer, a combination of these materials or other suitable material.

In the embodiment shown in FIG. 3A, linear movement of the actuating piston 242 within the inner cavity of the connection link 241 can cause the first and second claw members or grasper members 244a-b to open and close. The opening and closing of the first and second claw members or grasper members 244a-b may be in response to a tension applied to an actuating cable 420 coupled to the actuating piston 242. The actuating cable 420 may include one or more selected from the group consisting of: a metal cable, a plastic cable, a sold wire cable, a braided cable and a stainless steel wire braided cable.

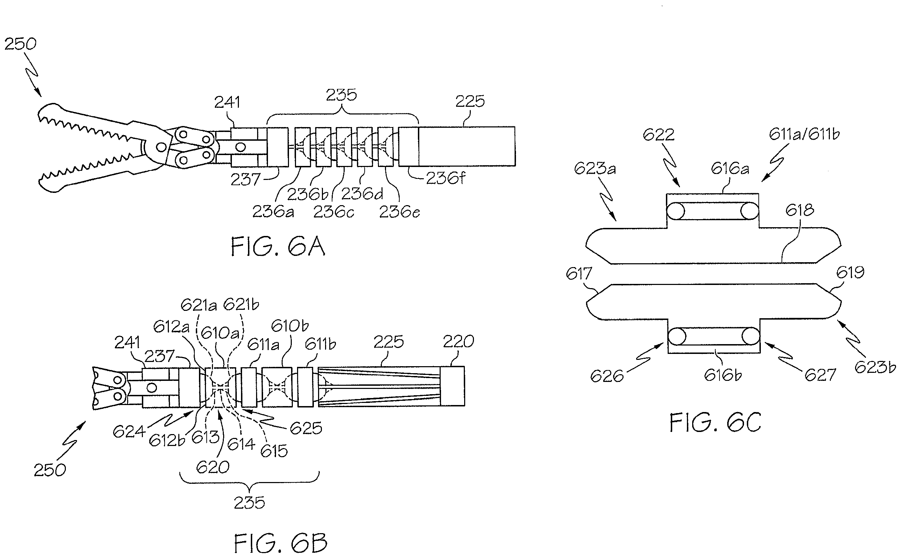

Although the articulation region 235 of the surgical tool 200 shown in FIGs. 3A and 3B illustrate a plurality of segment links 236, 237 having convex body portions 312, 352 (e.g., semi-ellipsoidal body portions, semi-spherical body portions) being oriented in a directing facing away from the functional element 250, the surgical tool 200 can be configured to include an articulation region 235 having a plurality of segment links 236, 237 having convex body portions 312, 352 (e.g., semi-ellipsoidal body portions, semi-spherical body portions) oriented in a direction facing the functional element 250 as shown in FIG. 6A.

In some embodiments, alternating convex body portions 312, 352 of the segment links 236, 237 may be constructed and arranged to have different coefficients of friction when mated with corresponding concave cavity portions of adjacent segment links 236. For example, alternating convex body portions 312, 352 of the segment links 236, 237 may include different materials and/or coatings to adjust and/or alter the coefficient of friction when mated with corresponding concave cavity portions of adjacent segment links 236.

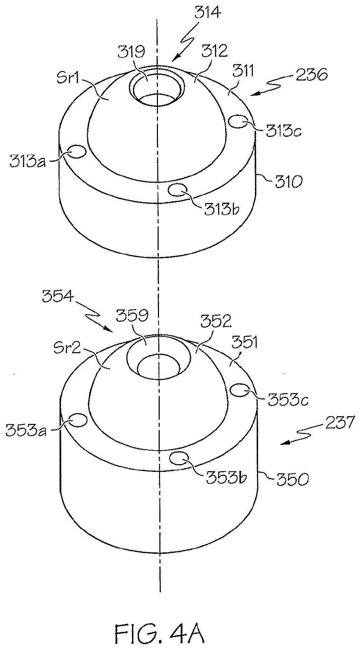

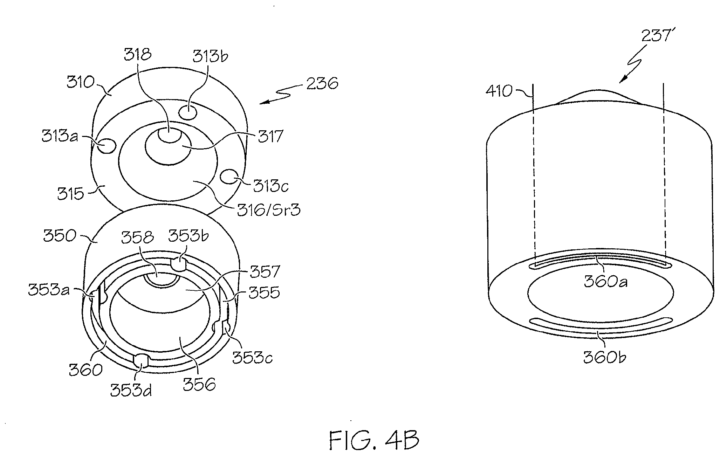

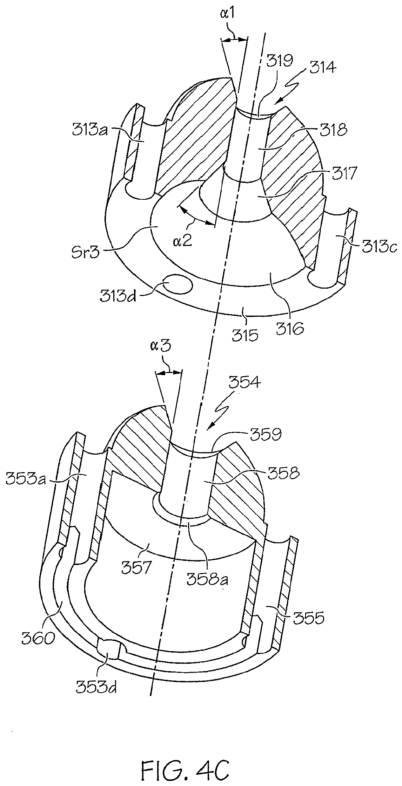

**FIG. 4A** is a top perspective view of segment links of the articulating surgical tool illustrated at FIG. 3, **FIG. 4B** is a bottom perspective view of segment links of the articulating surgical tool illustrated at FIG. 3, and **FIG. 4C** is a cross-sectional perspective view of the segment links illustrated at FIG. 4B.

The first segment link 236, 236a-e may include a body having a first portion 310 and a second portion 312. The first portion 310 may include a cylindrical body portion or a body portion having an elliptical cross-section, and the second portion 312 may include a convex body portion, a semi-ellipsoidal body portion or a semi-spherical body portion.

In a case where the second portion 312 includes a semi-spherical body portion, the semi-spherical body portion may include an outer surface having a spherical radius SR1 ranging between 1/20 of an inch and 1/4 of an inch. For example, the spherical radius SR1 may be about 1/20 of an inch.

Referring to FIG. 3, a semi-spherical body portion of the first segment link 236 may mate with a semi-spherical cavity portion of the cable transitioning segment 225 and/or the tool shaft 215, 220.

Referring back to FIGs. 4A-4C, the first segment link 236, 236a-e may include at least one articulation cable channel 313, 313a-d. The at least one articulation cable channel 313, 313a-d may include a first opening in an upper surface 311 of the first portion 310 and a second opening in a bottom surface 315 of the first portion 310. For example, in the embodiments shown at FIGs. 4A-4C, the at least one articulation cable channel 313, 313a-d may comprise first through fourth articulation cable channels 313a-d that are spaced 90° apart around the circumference or perimeter of the first portion 310. The at least one articulation cable channel 313, 313a-d may also comprise first through fourth articulation cable channels 313a-d that are positioned 90° apart from one another along a common radial path relative to a center axis of the first portion 310.

The first segment link 236, 236a-e may include an actuation cable channel 314. The actuation cable channel 314 may include a first opening at a diametric midpoint of the semi-spherical body portion of the first segment 236, 236a-e and a second opening at a diametric midpoint of the first portion 310 of the first segment 236, 236a-e.

The actuation cable channel 314 may include an upper taper 319 joined at the first opening that conforms the first opening with a cylindrical cavity 318 of the body of the first segment 236, 236a-e. The upper taper includes a draft angle α₁, which can range between 0° and 45°. The cylindrical cavity 318 may join a lower taper 317 of the body of the first segment 236, 236a-e. The lower taper 317 may conform the cylindrical cavity 318 with a concave cavity or a semi-spherical cavity 316 of the body of the first segment 236, 236a-e. The lower taper includes a draft angle α₂, which can range between 0° and 45°. The actuation cable channel 314 may include an upper taper 319 and/or a lower taper 317 to prevent pinching of an actuation cable 420 positioned within the actuation cable channel 314 during articulation states of the articulation region 235 of the surgical tool 200.

The semi-spherical cavity 316 of the body of the first segment 236, 236a-e may include an inner surface having a spherical radius SR3 ranging between 1/20 of an inch and 1/4 of an inch. For example, the spherical radius SR3 may be about 1/20 of an inch. The spherical radius SR3 may be substantially similar to or greater than a spherical radius SRI, SR2 of the first and second segment links 236, 237 so that a semi-spherical body portion of one of the first and second segment links 236, 237 can mate with a semi-spherical cavity portion of another first segment link.

The second segment link 237 may include a body having a first portion 350 and a second portion 352. The first portion 350 may include a cylindrical body portion or a body portion having an elliptical cross-section, and the second portion 352 may include a convex body portion, a semi-ellipsoidal body portion or a semi-spherical body portion.

In the case where the second portion includes a semi-spherical body portion, the semi-spherical body portion may include an outer surface having a spherical radius SR2 ranging between 1/20 of an inch and 1/4 of an inch. For example, the spherical radius SR2 may be about 1/20 of an inch.

Referring to FIG. 3A, the semi-spherical body portion of the second segment link 237 may mate with a semi-spherical cavity portion 316 of the first segment link 236.

Referring back to FIGs. 4A-4C, the second segment link 237 may include at least one articulation cable channel 353, 353a-d. The at least one articulation cable channel 353, 353a-d may include a first opening in an upper surface 351 of the first portion 350 and a second opening in a bottom surface 355 of the first portion 350. For example, in the embodiments shown at FIGs. 4A-4C, the at least one articulation cable channel 353, 353a-d may comprise first through fourth articulation cable channels 353a-d that are spaced 90° apart around the circumference or perimeter of the first portion 350. The at least one articulation cable channel 353, 353a-d may also comprise first through fourth articulation cable channels 353a-d that positioned 90° apart from one another along a common radial path relative to a center axis of the first portion 350.

Referring to FIG. 3A, at least two articulation cable channels 313, 313a-d of the first segment link 236, 236a-e may be aligned with at least two articulation cable channels 353, 353a-d of the second segment link 237 so as to provide a cable channel for the insertion of one or more articulation cables 410.

Referring back to FIGs. 4A-4C, the second segment 237 may include an actuation cable channel 354. The actuation cable channel 354 may include a first opening at a diametric midpoint of the semi-spherical body portion of the second segment 237 and a second opening at a diametric midpoint of the first portion 350 of the second segment 237.

The actuation cable channel 354 may include an upper taper 359 joined at the first opening that conforms the first opening with a first cylindrical cavity 358 of the body of the second segment 237. The upper taper 359 includes a draft angle α₃, which can range between 0° and 45°. The first cylindrical cavity 358 may join a second cylindrical cavity 356 of the body of the second segment 237. The first cylindrical cavity 358 may include a bevel 358a at an interface of the first cylindrical cavity 358 and an upper surface 357 of the second cylindrical cavity 356. A diameter of the first cylindrical cavity 358 may be less than a diameter of the second cylindrical cavity 356.

The second segment 237' may further include at least one cavity slot 360, 360a-b formed in the bottom surface 355 of the first portion 350 of the second segment 237'. The at least one cavity slot 360, 360a-b may include a single continuous cavity slot 360 or may include a first cavity slot 360a and a second cavity slot 360b. The first cavity slot 360a may extend from a first articulation cable channel 353a of the at least one articulation cable channel to a second articulation cable channel 353b of the at least one articulation cable channel.

A first articulation cable 410 may be positioned within the first articulation cable channel 353a, the first cavity slot 360a and the second articulation cable channel 353b. The first articulation cable 410 may be secured to a surface of the first cavity slot 360a. For example, the first articulation cable may be welded to the surface of the first cavity slot 360a, glued to the surface of the first cavity slot 360a and/or press fit within the first cavity slot 360a.

The second cavity slot 360b may extend from a third articulation cable channel 353c of the at least one articulation cable channel to a fourth articulation cable channel 353d of the at least one articulation cable channel. A second articulation cable 410 may be positioned within the third articulation cable channel 353c, the second cavity slot 360b and the fourth articulation cable channel 353d.

The at least one cavity slot 360 may extend about an entire perimeter or circumference of the bottom surface 355 of the first portion 350 or cylindrical body portion of the second segment 237. In this manner, the second opening of the at least one articulation cable channel 353, 353a-d may be partially defined by the at least one cavity slot 360. At least one articulation cable 410 may be positioned within the at least one articulation cable channel 353, 353a-d, and may be secured to a surface of the at least one cavity slot 360.

The segment links 236, 237 may include a material selected from the group consisting of: metal, plastic, a thermoplastic polymer, stainless steel, polyvinyl chloride, a liquid-crystal polymer and polytetrafluoroethylene. The segment links 236, 237 may be rigid. The second segment link 237 may include a material different from that of the first segment link 236.

In some embodiments, a height of the second portions 312, 352 of the segment links 236, 236a-e, 237 may be different such that an angle of articulation between one or more segment links can be restricted to different angles of articulation. For example, a first segment link 236, 237 or a first group of segment links may be restricted to approximately 12° to 15° per segment, and a second segment link 236, 237 or a second group of segment links may be restricted to approximately 8° to 11° per segment.

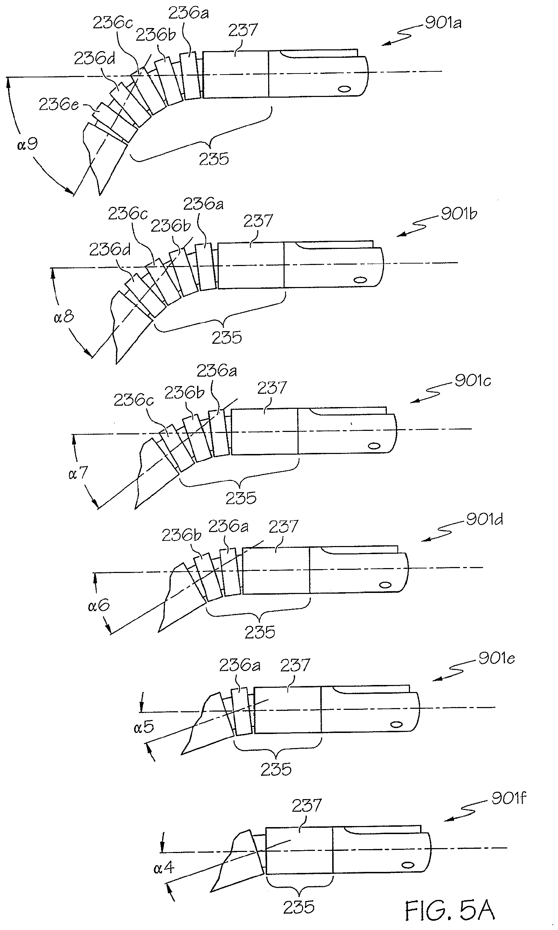

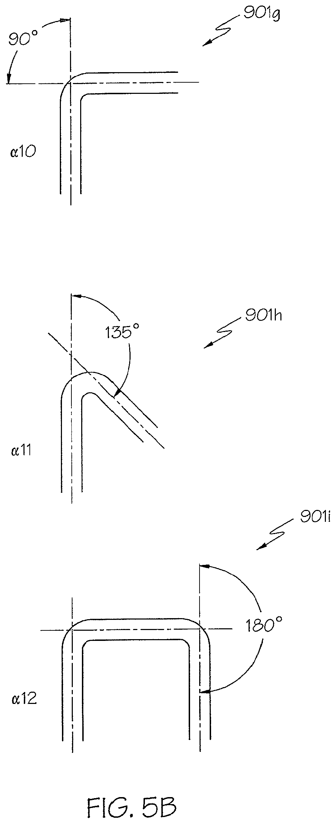

**FIGs. 5A** **and** **5B** are perspective views illustrating articulation ranges of the articulating surgical tool illustrated at FIG. 2. The articulation region 235 of the surgical tool 200 is illustrated in varying articulation states 901a-i.

As described above, the articulation region 235 of the tool shaft may include one or more segment links 236, 237. In embodiments including two or more segments links 236, 237, each segment link 236, 237 may be sequentially coupled. In this manner, a plurality of segment links 236, 237 may articulate with respect to one another.

The segment links 236, 237 of the articulation region may be constructed and arranged to restrict an angle of articulation. For example, a bottom surface of a first portion of a first segment link may abut an upper surface of a first portion of a second segment link to restrict an angle of articulation with respect to a center axis of each of the first and second segment links.

In some embodiments, the angle of articulation can be restricted to approximately 12° to 15° per segment 236, 237. For example, referring to the articulation state 901f, a surgical tool 200 including a single segment link 237 may be restricted to a maximum angle of articulation α₄ that ranges between approximately 12° to 15°. Referring to the articulation state 901e a surgical tool 200 including two segment links 236a, 237 may be restricted to a maximum angle of articulation α₅ that ranges between approximately 24° to 30°. Referring to the articulation state 901d, a surgical tool 200 including three segment links 236a-b, 237 may be restricted to a maximum angle of articulation α₆ that ranges between 36° to 45°. Referring to the articulation state 901c, a surgical tool 200 including four segment links 236a-c, 237 may be restricted to a maximum angle of articulation α₇ that ranges between approximately 48° to 60°. Referring to the articulation state 901b, a surgical tool 200 including five segment links 236a-d, 237 may be restricted to a maximum angle of articulation α₈ that ranges between approximately 60° to 75°. Referring to the articulation state 901a, a surgical tool 200 including six segment links 236a-e, 237 may be restricted to a maximum angle of articulation α₉ that ranges between approximately 72° to 90°. Referring to the articulation state 901g, a surgical tool 200 including seven segment links may be restricted to a maximum angle of articulation α₁₀ that ranges between approximately 84° to 105°. Referring to the articulation state 901h, a surgical tool 200 including nine segment links may be restricted to a maximum angle of articulation α₁₁ that ranges between approximately 108° to 135°. Referring to the articulation state 901i, a surgical tool 200 including twelve segment links may be restricted to a maximum angle of articulation α₁₂ that ranges between approximately 144° to 180°. Accordingly, an articulation state of the surgical tool 200 including n segment links may be restricted to a maximum angle of articulation α that ranges between approximately (12*n)° to (15*n)°.

Referring to FIGs. 6A, 6B, 7, 8A-8E and 9, the alternative segment link configurations illustrated therein may be readily incorporated into the articulating surgical tool 200 shown in FIGs. 2 and 3. For example, any one of the articulation regions 235 illustrated in FIGs. 6A, 6B, 7, 8A-8E and 9 may replace the articulation region 235 shown in FIGs. 2 and 3.

**FIG. 6A** is a side perspective view illustrating an alternative segment link configuration of an articulating surgical tool. As described above, the surgical tool 200 can be configured to include an articulation region 235 having a plurality of segment links 236, 237 having convex body portions 312, 352 (e.g., semi-ellipsoidal body portions, semi-spherical body portions) oriented in a direction facing the functional element 250 as shown in FIG. 6A. The segment links 236, 237 shown in FIG. 6A may be substantially similar to the segment links 236, 237 shown in FIGs. 3 and 4A-4C and are indicated as having like reference characters.

**FIG. 6B** is a side perspective view illustrating an alternative segment link configuration of an articulating surgical tool, and **FIG. 6C** is a sectional view of the third segment links illustrated in FIG. 6B. The articulation region 235 of the surgical tool 200 may include a first segment link 237, one or more second segment links 610a-b, and one or more third segment links 611a-b. For example, in the embodiment shown in FIG. 6B, the articulation region 235 includes a first segment link 237, two (2) second segment links 610a-b and two (2) third segment links 611a-b.

The first segment link 237 may be similar to the distal segment link 237 shown in FIGs. 4A-4C, and may be coupled to the functional element 250. However, as described above, the articulation region 235 may be provided at any position between the functional element 250 and the proximal end 215p of the tool shaft 215 (see for example FIG. 2).

At least one second segment link 611a-b may be coupled directly or indirectly to the tool shaft 215, 220. For example, the second segment 611b may be coupled to the second tool shaft 220 via the cable transitioning segment 225, which may distribute multiple cables (e.g., one or more actuating cables 420 and/or one or more articulation cables 410) from the tool shaft 215, 220 to channels 612a-b, 616a-b of the segment links 610a-b, 611a-b, 237.

At least one third segment link 611a-b may be coupled between the first segment link 237 and one of the second segment links 610a-b. For example, in the embodiment shown in FIG. 6B, the third segment link 611a is coupled between the first segment 237 and the second segment link 610a, and the third segment link 611b is coupled between the second segment link 610a and the second segment link 610b.

The second segment link 610a-b may include a body 620 having first and second concave cavities 621a-b formed at opposite end surfaces of the body 620. The first and second concave cavities 621a-b may include semi-ellipsoidal cavities or semi-spherical cavities. In an embodiment having semi-spherical cavities, the semi-spherical cavities may have spherical radii that match spherical radii of semi-spherical body portions of the third segment links 611a-b.

The first concave cavity 621a may join a first taper 613 of the body 620 of the second segment link 610a-b, and the first taper 613 may conform the first concave cavity 621a to a first opening of a cylindrical cavity 614. The second concave cavity 621b may join a second taper 615 of the body 620 of the second segment link 610a-b, and the second taper 615 may conform the second concave cavity 621b to a second opening of the cylindrical cavity 614. In this manner, an actuation cable channel may be formed within the body 620 of the second segment link 610a-b, extending from the first concave cavity 621a to the second concave cavity 621b. In addition, the first and second tapers 613, 615 may prevent pinching of an actuation cable 420 positioned within the actuation cable channel of the second segment link 610a-b during articulation states of the articulation region 235.

The third segment link 611a-b may include a body having a first convex body portion 623a, a second body portion 622, and a third convex body portion 623b. The first and third body portions 623a-b may include semi-ellipsoidal body portions or semi-spherical body portions, and the second body portion 622 may include a cylindrical body portion.

The third segment link 611a-b may include a first taper 617 joined at a first opening in the first convex body portion 623a. The first taper 617 may conform the first opening in the first convex body portion 623a to a cylindrical cavity 618 of the third segment 611a-b. The third segment link 611a-b may include a second taper 619 joined at a second opening in the second convex body portion 623b. The second taper 619 may conform the second opening in the second convex body portion 623b to the cylindrical cavity 618 of the third segment 611a-b. In this manner, an actuation cable channel may be formed within the body of the third segment link 611a-b, extending from the first opening in the first convex body portion 623a to the second opening in the second convex body portion 623b. In addition, the first and second tapers 617, 619 may prevent pinching of an actuation cable 420 positioned within the actuation cable channel of the third segment link 611a-b during articulation states of the articulation region 235.

As described above with reference to the segment links 236, 237 shown in FIGs. 4A-4C, the second and third segment links 610a-b, 611a-b may likewise include at least one articulation cable channel 612a-b, 616a-b. The at least one articulation cable channel 612a-b, 616a-b may include a first opening in a first surface 624, 626 of the bodies of the second and third segment links 610a-b, 611a-b, and a second opening in a second surface 625, 627 of the bodies of the second and third segment links 610a-b, 61 1a-b. For example, in the embodiments shown at FIG. 6B, the at least one articulation cable channel of the second and third segment links 610a-b, 611a-b may comprise first through fourth articulation cable channels that are spaced 90° apart around the circumference or perimeter of the bodies of the second and third segment links 610a-b, 61 1a-b. The at least one articulation cable channel may also comprise first through fourth articulation cable channels that are positioned 90° apart from one another along a common radial path relative to a center axis of the second and third segment links 610a-b, 611a-b.

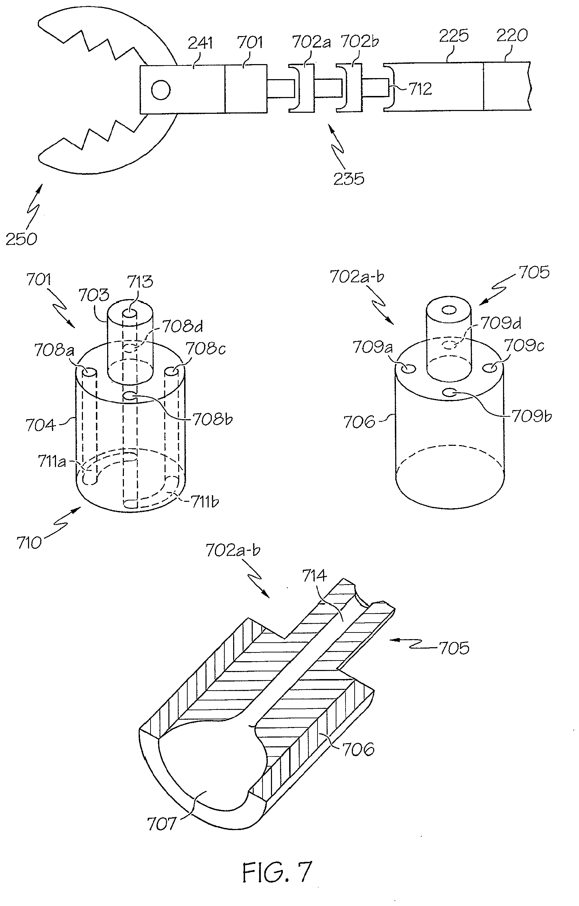

**FIG. 7** is a perspective view illustrating an alternative segment link configuration of an articulating surgical tool. The articulation region 235 of the surgical tool 200 may include a first segment link 701 and one or more second segment links 702a-b. For example, in the embodiment shown in FIG. 7, the articulation region 235 includes a first segment link 701 and two (2) second segment links 702a-b.

The first segment link 701 may include a body 704 and a protrusion 703. The body 704 may include a body having an elliptical cross-section or a cylindrical body, and the protrusion 703 may include an elliptical protrusion or a cylindrical protrusion. For example, in the embodiment shown in FIG. 7, the first segment link 701 is shown having a cylindrical body and a cylindrical protrusion. A diameter of the body 704 may be greater than a diameter of the protrusion 703.

The first segment link 701 may include at least one cavity slot 711a-b formed in a bottom surface 710 of the body 704. The at least one cavity slot 711a-b may be similar to the at least one cavity slot 360, 360a-b formed in the bottom surface 355 of the first portion 350 of the second segment 237 shown in FIG. 4B. The at least one cavity slot may include a single continuous cavity slot (not shown), such as the single continuous cavity slot 360 shown in FIG. 4B or may include a first cavity slot 711a and a second cavity slot 711b as shown in FIG. 7.

The second segment link 702a-b may include a body 706 and a protrusion 705. The body 706 may include a body having an elliptical cross-section or a cylindrical body, and the protrusion 705 may include an elliptical protrusion or a cylindrical protrusion. For example, in the embodiment shown in FIG. 7, the second segment link 702a-b is shown having a cylindrical body and a cylindrical protrusion. A diameter of the body 706 may be greater than a diameter of the protrusion 705.

The second segment link 702a-b may include at least one concave cavity 707. The concave cavity may include a semi-ellipsoidal cavity or a semi-spherical cavity. In this manner, a protrusion 703, 705 of the first and second segment links 701, 702a-b may mate with a concave cavity 707 of another second segment link 702a-b. For example, in the embodiment shown in FIG. 7, the protrusion 703 of the first segment link 701 is shown mated with the concave cavity 707 of the second segment link 702a, and the protrusion 705 of the second segment link 702a is shown mated with the concave cavity 707 of the second segment link 702b. In this example, the cable transitioning segment 235 includes a concave cavity 712, which is shown mated with a protrusion 705 of the second segment link 702b.

As described above with reference to the segment links 236, 237 shown in FIGs. 4A-4C, the first and second segment links 701, 702a-b may likewise include at least one articulation cable channel 708a-d, 709a-d. The at least one articulation cable channel 708a-d, 709a-d may include a first opening in a first surface of the bodies 704, 706 of the first and second segment links 701, 702a-b, and a second opening in a bottom surface of the bodies 704, 706 of the first and second segment links 701, 702a-b. For example, in the embodiments shown at FIG. 7, the at least one articulation cable channel of the first and second segment links 701, 702a-b may comprise first through fourth articulation cable channels 708a-d, 709a-d that are spaced 90° apart around the circumference or perimeter of the bodies 704, 706 of the first and second segment links 701, 702a-b. The at least one articulation cable channel may also comprise first through fourth articulation cable channels 708a-d, 709a-d that are positioned 90° apart from one another along a common radial path relative to a center axis of the first and second segment links 701, 702a-b.

The first and second segment links 701, 702a-b may include actuation cable channels 713, 714. The actuation cable channels 713, 714 may include a first opening at a diametric midpoint of the protrusions 703, 705 and a second opening at a diametric midpoint of the bottom surfaces of the bodies 704, 706. Although not shown, the first and second openings may join first and second tapers that conform the first and second openings to a cylindrical cavity so as to form a channel. As described above, the tapers may prevent pinching of an actuation cable 420 positioned within the actuation cable channels 713, 714 of the segment links 701, 702a-b during articulation states of the articulation region 235.

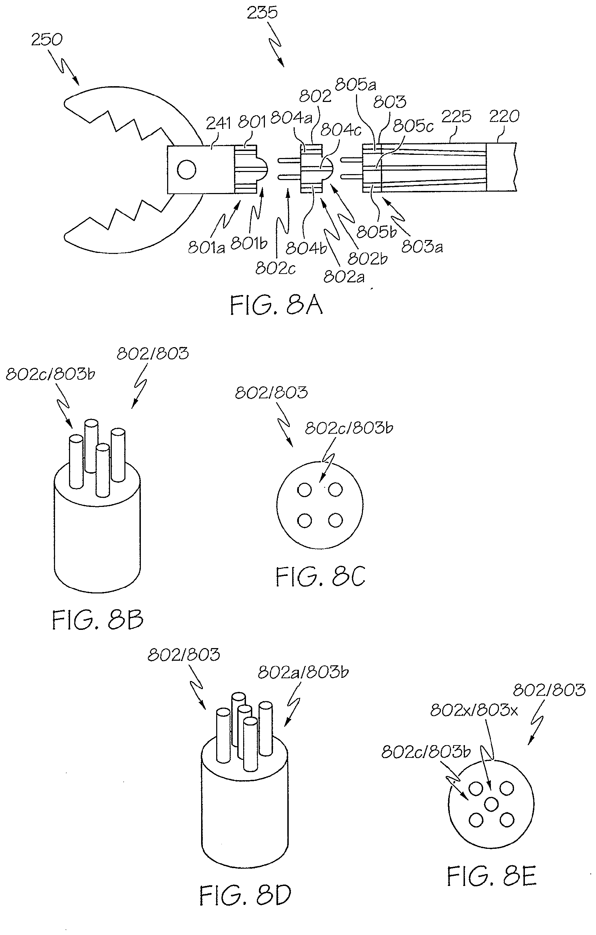

**FIG. 8A** is a perspective view illustrating an alternative segment link configuration of an articulating surgical tool. The articulation region 235 of the surgical tool may include a first segment link 801, one or more second segment links 802, and a third segment link 803.

The first segment link 801 may include a body having a first body portion 801a and a second body portion 801b. The first body portion 801a may include a body portion having an elliptical cross-section or a cylindrical body portion, and the second body portion 801b may include a convex body portion, a semi-ellipsoidal body portion or a semi-spherical body portion. The first segment link may be similar to the distal segment link 237 shown in FIGs. 4A-4C.

The second segment links 802 may include a first body portion 802a, a second body portion 802b, and a plurality of protruding posts 802c extending from a surface of the first body portion 802a. The first body portion 802a may include a body portion having an elliptical cross-section or a cylindrical body portion, and the second body portion 802b may include a convex body portion, a semi-ellipsoidal body portion or a semi-spherical body portion. The posts 802c may include cylindrically shaped posts, and may have rounded or beveled top surfaces.

The third segment link 803 may include a first body portion 803a and a plurality of protruding posts 802c extending from a surface of the third body portion 803a. The posts 803b may include cylindrically shaped posts, and may have rounded or beveled top surfaces.

The second body portion 801b of the first segment 801 may mate with the plurality of posts 802c of the second segment 802, and the second body portion 802b of the second segment link 802 may mate with the plurality of posts 803b of the third segment 803. In this manner, friction may be reduced at the interface between the second body portion 801b of the first segment link 801 and the posts 802c of the second segment link 802, and the interface between the second body portion 802b of the second segment link 802 and the posts 803b of the third segment link 803.

As described above with reference to the segment links 236, 237 shown in FIGs. 4A-4C, the second and third segment links 802, 803 may likewise include at least one articulation cable channel 804a-b, 805a-b. For example, in the embodiments shown at FIG. 8A, the at least one articulation cable channel of the second and third segment links 802, 803 may comprise first through fourth articulation cable channels that are spaced 90° apart around the circumference or perimeter of the bodies 802a, 803a of the second and third segment links 802, 803. The at least one articulation cable channel may also comprise first through fourth articulation cable channels that are positioned 90° apart from one another along a common radial path relative to a center axis of the second and third segment links 802, 803.

The second and third segment links 802, 803 may include actuation cable channels 804c, 805c. The actuation cable channels 804c, 805c may be positioned at a diametric midpoint of the second and third segment links 802, 803.

FIGs. 8B-8E illustrate alternative post configurations in accordance with the alternative segment link configuration shown in FIG. 8A.

**FIG. 8B** is a perspective view of a segment link, and **FIG. 8C** is a top view of the segment link illustrated in FIG. 8B. As described above, the second and third segment links 802, 803 may include a plurality of posts 802c, 803b. The plurality of posts 802c, 803b may be arranged along a common radial path relative to a center axis of the second and third segment links 802, 803, and may be spaced apart by a common distance. For example, in the embodiment shown in FIGs. 8B and 8C, the plurality of posts 802c, 803b include first through forth posts. The plurality of posts 802c, 803b may have a common height.

**FIG. 8D** is a perspective view of a segment link, and **FIG. 8E** is a top view of the segment link illustrated in FIG. 8D. As described above, the second and third segment links 802, 803 may include a plurality of posts 802c, 803b. The plurality of posts 802c, 803b may be arranged along a common radial path relative to a center post 802x, 803x of the second and third segment links 802, 803, and may be spaced apart by a common distance. For example, in the embodiment shown in FIGs. 8D and 8E, the plurality of posts 802c, 803b include first through fourth posts. The first through fourth posts are arranged along a common radial path relative to the center post 802x, 803x. In this exemplary configuration, the plurality of posts 802c, 803b arranged about the center posts 802x, 803x and may each have a first height greater than a second height of the center post 802x, 803x.

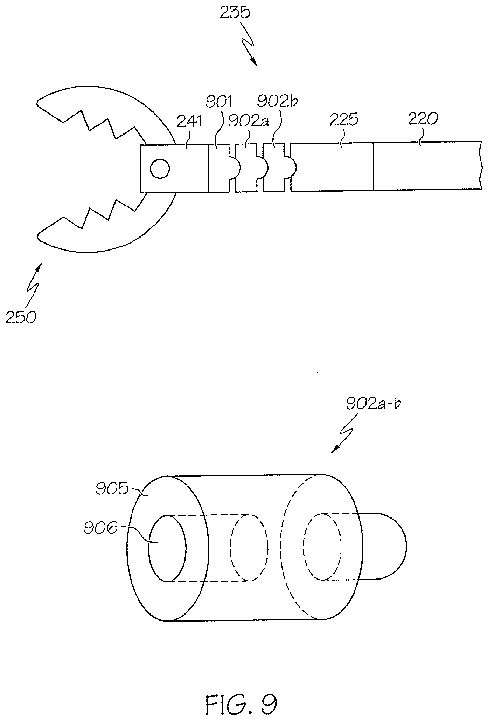

**FIG. 9** is a perspective view illustrating alternative segment links of an articulating surgical tool. The articulation region 235 of the surgical tool may include a first segment link 901 and one or more second segment links 902a-b.

The first segment link 901 may be similar to the distal segment link 237 shown in FIGs. 4A-4C, and the second segment links 902a-b may be similar to the first segment link 236 shown in FIGs. 4A-4C. However, instead of including a concave cavity 316 as shown in FIGs. 4A-4C, the second segment links 902a-b may include an elliptical or circular opening 906 formed in the bottom surface 905 of the second segment links 902a-b. The opening 906 may have a diameter less than twice the spherical radius of the convex body portions of the first and second segments 901, 902a-b so that when mated, the convex body portions of the first and second segment 901, 902a-b partially protrude within the opening 906.

As described herein, tension or slack applied to the articulation cables can permit an operator to change the articulation state of the surgical tool. For example, an operator can apply a force to the articulation cables to bend the surgical tool to a desired angle of articulation. The operator can maintain the angle of articulation until a different force is applied to the articulation cables. Continuing with this example, the operator can apply tension or slack to the actuation cable, for example, to open and close a grasper, while the tool shaft is maintained at the angle of articulation. The operator can alternatively open and close the grasper while also bending the surgical tool. However, if too much tension is applied to the actuation cable, the excess force applied to the actuation cable can inadvertently cause the articulation cables to bind and/or cause the segment links of the articulation region to enter a lock state, for example, by binding together. Thus, it is desirable that the forces imparted on an actuation cable and the forces imparted on the articulation cables be applied independently of one another. For example, when tension or slack is applied to the actuation cable in order to operate a grasper or other functional element, it is desirable that a force generated by the movement of the actuation cable does not affect articulation cables, or the segment links of the articulation region.

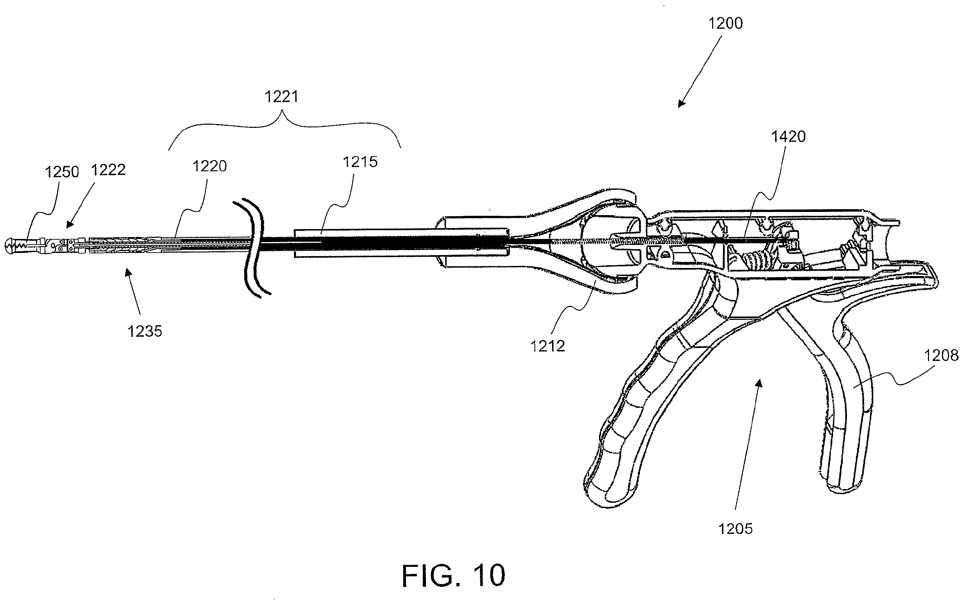

**FIG. 10** is a cross-sectional side view of an articulating surgical tool 1200, in accordance with another embodiment of the present inventive concepts. The surgical tool 1200 of FIG. 10 can prevent inadvertent binding or locking from occurring during operation. The surgical tool 1200 can be implemented in a system for performing a medical procedure, for example, the system 100 described above. Accordingly, the surgical tool 1200 can be part of an articulating probe, for example, the articulating probe 120 described herein, which can guide the surgical tool 1200 and/or other surgical tools 200, 200a-d described in abovementioned embodiments within a patient body.

The surgical tool 1200 can be constructed and arranged to be controlled via a human interface device, for example, a haptic controller, a joystick, a track ball, a mouse, or an electromechanical or processor-based device.

The surgical tool 1200 can include a tool handle 1205, a first assembly 1221, and a second assembly 1222. The first assembly 1221 and the second assembly 1222 can be co-located at an articulating probe, for example, co-located in a lumen of an articulating probe.

In an embodiment, the tool handle 1205 is coupled to a proximal end of a support element, for example support element 1231 described below, at a proximal end of the first assembly 1221. The tool handle 1205 can include one selected from the group consisting of: scissor handles, a palm-held grip, a thumb/index/middle finger grip and a pistol grip. The tool handle 1205 can include a trigger 1208 that applies a force for the movement of elements of the second assembly 1222, such as an activation element 1420.

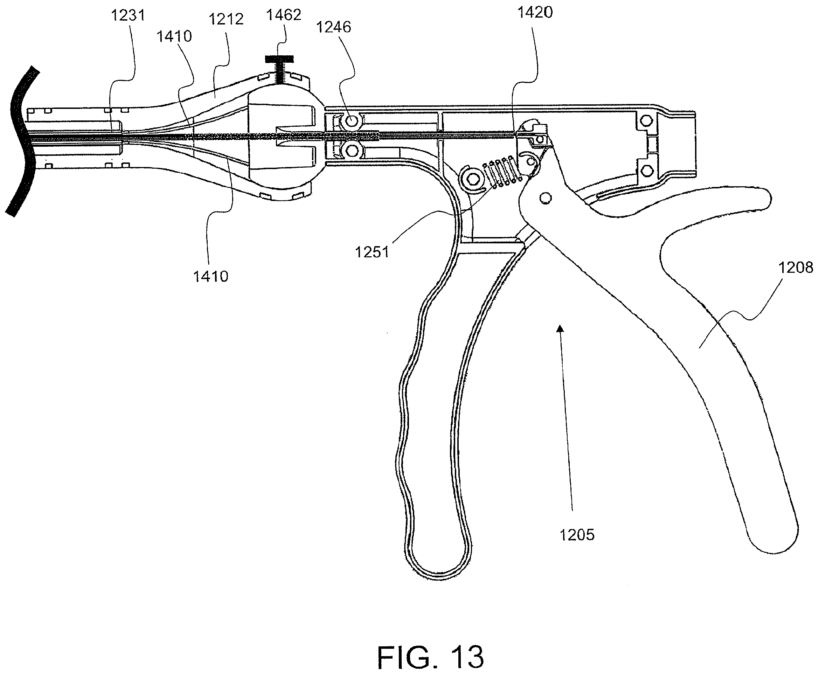

In an embodiment, the surgical tool 1200 includes a ball and socket mechanism 1212 that is constructed and arranged to manipulate or otherwise control functions and movement of elements of the surgical tool 1200. Although not shown in FIG. 10, one or more steering cables, also referred to as articulation cables, may be secured to the ball and socket mechanism 1212, shown as articulation cables 1410 at FIG. 13. One or more activation elements 1420 may be secured to the tool handle 1205, shown in greater detail at FIG. 13. A movement of the ball and socket mechanism 1212 can provide tension or slack on one or more of the steering cables 1410 secured to the ball and socket mechanism 1212, thereby adjusting an articulation state of an articulation region 1235 of the first assembly 1221. The articulation region 1235 can be at a distal end of the first assembly 1221 proximal to the second assembly 1222. The articulation region 1235 can alternatively be located anywhere along the first assembly 1221 between the distal end of the first assembly 1221 and a proximal end of the first assembly 1221. Forces related to tension, slack, and the like can be applied by the activation element 1420 in response to a movement of the handle 1205, for example, squeezing the trigger 1208.

The first assembly 1221 of the surgical tool 1200 can include a first tool shaft 1215 and a second tool shaft 1220. A proximal end of the first tool shaft 1215 can be coupled to the tool handle 1205, for example, via the ball and socket mechanism 1212. In an embodiment, a distal end of the first tool shaft 1215 is coupled to a proximal end of the second tool shaft 1220. A distal end of the second tool shaft 1220 may be directly coupled to the articulation region 1235 or can be otherwise in communication with the articulation region 1235 via intervening components between the second tool shaft 1220 and the articulation region 1235, for example, a cable transitioning segment the same as or similar to the segment 225 described above.

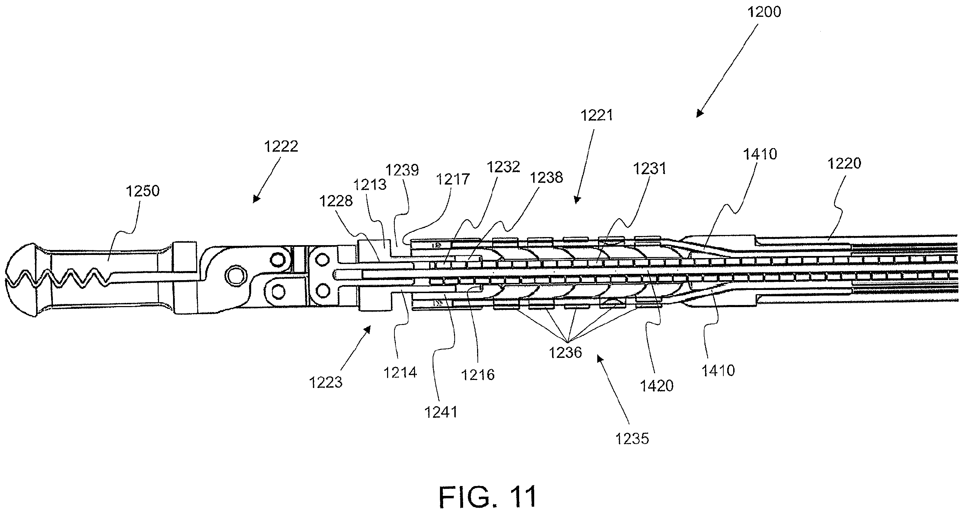

Although the articulation region 1235 is shown at a distal end of the surgical tool 1200, the articulation region 1235 may be provided at any position between a functional mechanism 1250, also referred to as a functional element, and the proximal end of the first tool shaft 1215. Details of the articulation region 1235 are described herein with reference to FIG. 11.

The first tool shaft 1215 can include a rigid tool shaft and the second tool shaft 1220 can include a flexible tool shaft. Alternatively, the tool shafts 1215, 1220 of the surgical tool 1200 can each include rigid and/or flexible tool shafts.

At least one of the tool shafts 1215, 1220 may include a lumen guiding member (not shown) having at least one cable channel, for example, a first cable channel for receiving the activation element 1420 and at least one second cable channel for receiving steering cables 1410. The first cable channel is preferably larger in diameter than the articulation cable channel 226 described with reference to FIG. 2-9 to accommodate elements of the second assembly 1222 such as a support element 1231, described below. In the other embodiments, only the activation element 1420 extends through the articulation segments. Here, the second cable channel for the steering cables 1410 has smaller dimensions such as width or diameter than the first cable channel. The first cable channel can be positioned at a diametric midpoint of the tool shaft, and the at least one second cable channel can be positioned along a circumference or perimeter of the tool shaft. For example, the lumen guiding member may include a multi-lumen stiffening rod, for example, a five lumen stiffening rod having a first cable channel and four second cable channels. The lumen guiding member may include a material selected from the group consisting of: metal, plastic, a thermoplastic polymer, stainless steel, polyvinyl chloride, a liquid-crystal polymer, polytetrafluoroethylene, and a combination of these materials or other suitable material.

The surgical tool 1200 can include a locking device (not shown) that is constructed and arranged to lock an articulated position of the functional mechanism 1250. The surgical tool may further include a locking device that is constructed and arranged to lock an operational mode of the functional mechanism 1250. For example, the locking device can be constructed and arranged to lock the articulation region 1235 in place to maintain a particular state, for example, an angle, of the surgical tool 1200 and/or maintain a grasping state of the functional mechanism 1250 (e.g., opened, closed, partially closed).

**FIG. 11** is a detailed view of the articulating surgical tool 1200 illustrated in FIG. 10, in accordance with another embodiment of the present inventive concepts.

The second assembly 1222 comprises an elongated support element 1231, an elongated activation element 1420, and a functional mechanism 1250. The second assembly 1222 is in communication with the first assembly 1221 so that the support element 1231 can be movable relative to the first assembly 1221. In an embodiment, the support element 1231 in a first state extends along an axis, and is constructed and arranged for flexibility in a radial direction relative to the axis, while maintaining column strength when a force is applied to the support element 1231 in an axial direction. In a second state, the support element 1231 can flex or bend relative to the axis. The support element 1231 can include elastic bending and/or plastic deformation characteristics for transitioning between the first and second states.

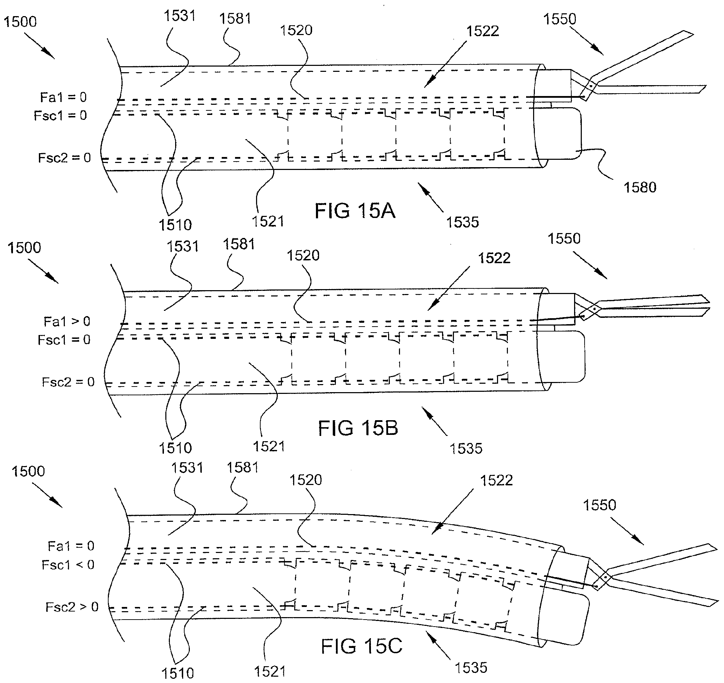

The support element 1231 can be constructed and arranged as a coil, rod, hollow tube, a linear arrangement of multiple links, or related structure. The support element 1231 can have a cross-section of any well-known suitable shape, including but not limited to a circle, oval, polygon, square, triangle, or a rectangle. The support element 1231 includes a lumen that extends along a direction of extension of the support element 1231. The activation element 1420 is positioned in the lumen, and can move relative to the support element 1231, for example, along the direction of extension of the support element 1231. In one embodiment, the activation element 1420 moves freely within the support element 1231. In another embodiment, an activation element 1520 moves freely proximal to an outer surface of the support element 1531, as shown at FIGs. 15A-15C.

The support element 1231 has limited compression in the direction of its extension, but is flexible in a lateral direction, or transverse direction, relative to the direction of extension. For example, during operation, the support element 1231 can bend in a same or similar direction as the first assembly 1221. Here, the activation element 1420 can move along the direction of the bend in the support element 1231 independently of any movement in the first assembly 1221. Thus, when a force is applied by the activation element 1420 to the functional mechanism 1250, for example, pulling the activation element in a direction away from the functional mechanism 1250 during operation of the functional mechanism 1250, the support element 1231 maintains a rigid position during movement of the activation element 1420 in the lumen of the support element 1231. In other words, the support element 1231 can absorb a load caused by the force imparted by the movement of the activation element 1420. More specifically, the support element 1231 provides rigidity in a longitudinal direction, while also providing some flexibility in a lateral direction when a force is a applied which prevents binding, but still allows the tool to bend with a probe or the like. The support element 1231 can therefore prevent or otherwise reduce an amount of a force from being transferred to the first assembly 1221 when the force is imparted by the movement of the activation element 1420. As such, the force imparted by the movement of the activation element 1420 can be isolated from the first assembly. In cases where such isolation of the forces is not absolute, perfect, or complete, a marginal amount of force transfer to elements of the first assembly 1221 can occur. However, any transfer of force is reduced to nevertheless preventing or largely mitigate any undesirable effects on the first assembly 1221, for example, binding of steering cables or inadvertent locking of links at the articulation region 1235.

The activation element 1420 can be constructed and arranged as a wire, cable, fiber, string, and the like for operating the functional mechanism 1250. The activation element 1420 can be formed of polytetrafluoroethylene, also referred to as Teflon®, graphite, metal, plastic, or other material permitting operation of the functional mechanism 1250. In some embodiments, activation element 1420 may comprise a wire or optical fiber configured to deliver energy or data. The energy can be mechanical energy, electrical energy, radiation energy, or other form of energy known to those of ordinary skill in the art. In some embodiments, activation element 1420 may comprise at least a portion of its outer surface that is lubricious, such as a surface portion comprising a material selected from the group consisting of: Teflon; graphite; a hydrophilic coating; a surface area reducing texture; and combinations of these.

The functional mechanism 1250 can be directly or indirectly coupled to the activation element 1420, for example, via a linkage mechanism at the functional mechanism 1250. In this manner, a movement of the functional mechanism 1250 can occur in response to a movement of the activation element 1420. The structure, location, and function of the functional mechanism 1250 can be similar to the functional element 250 described above. Details of the functional mechanism 1250 are therefore not repeated for brevity. The functional mechanism 1250 can be a grasper; a scissor; a reciprocating cutter; a claw, a cutter, a knife, or other tool well-known to those of ordinary skill in the art used for performing medical procedures. The functional mechanism 1250 can be constructed and arranged to articulate with respect to a direction of extension of the first assembly 1221.

The functional mechanism 1250 can include an actuation piston (not shown) coupled to the activation element 1420 to link the activation element 1420 to the functional mechanism 1250. The structure, location, and function of the actuation piston can be similar to the actuation piston 242 described herein. Details of the actuation piston are therefore not repeated for brevity.

The second assembly 1222 can also comprise a mount such as a clevis 1223 that is coupled to the support element 1231. The clevis 1223 can be coupled to a distal end 1232 of the support element 1231. An inner surface of the clevis 1223 can be coupled to an outer surface of the support element 1231 at the distal end 1232 of the support element 1231. In an embodiment, the clevis 1223 is bonded to the support element 1231, for example, using an adhesive. In another embodiment, the clevis 1223 is welded to the support element 1231. In other embodiments, the clevis 1223 and the support element 1231 are in communication by swaging, threading, pinning, snap-fitting, press-fitting, or coupling together in a well-known manner to those of ordinary skill in the art.

The clevis 1223 can include a base 1213 and a protrusion 1214 extending from the base 1213. In an embodiment, the base 1213 is wider than the protrusion 1214. The protrusion 1214 of the clevis 1223 can extend into an opening, or recess, at a connection link 1241 at a distal end of the first assembly 1221. In an embodiment, an outer width of the base 1213 is equal to an outer width of the distal end 1217 of the first assembly 1221. The protrusion 1214 can have a cylindrical outer surface. The opening at the connection link 1241 for receiving the clevis 1223 can have a cylindrical inner surface.

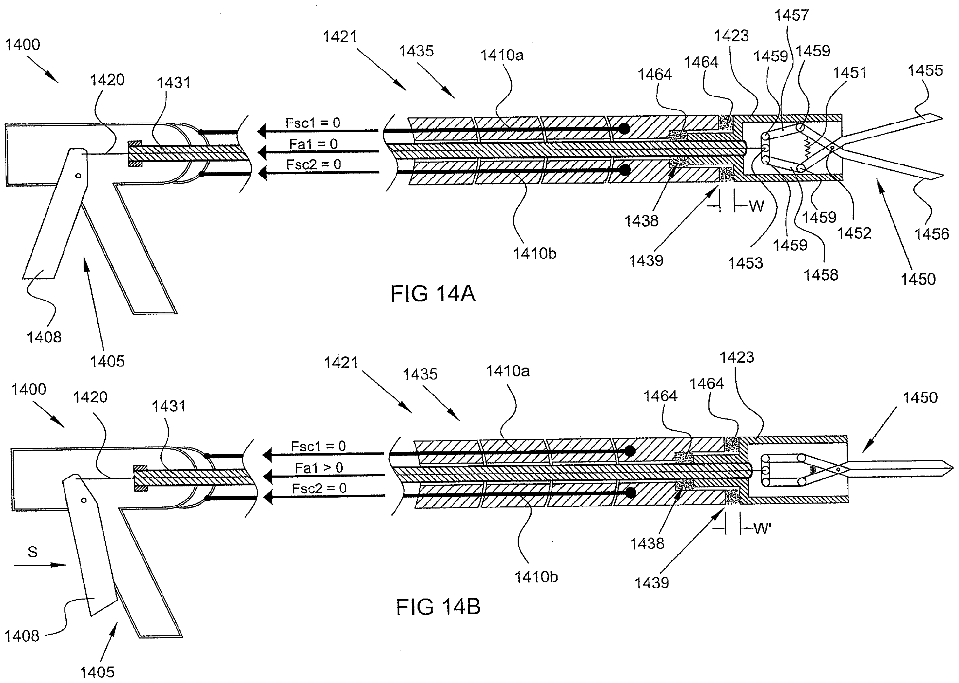

The clevis 1223 includes an opening 1228 for receiving the functional mechanism 1250. The activation element 1420 can be coupled to the functional mechanism 1250 and translation of the activation element 1420 operates the functional mechanism 1250. For example, translation of the activation element 1420 controls the movement of the functional mechanism 1250. The opening 1228 can be constructed and arranged to permit the functional mechanism 1250 to expand and contract relative to the opening 1228 during the movement of the functional mechanism 1250, for example, opening and closing a grasper as shown in FIGs. 14A and 14B.

The activation element 1420 moves freely along a direction of extension of the support element 1231 and the clevis 1223. The activation element 1420 can include one or more actuation cables, for example, similar to the actuation cables 420 described herein.

The surgical tool 1200 also includes a first longitudinal clearance 1238 and/or a second longitudinal clearance 1239 between the clevis 1223 and the distal link 1241 of the first assembly 1221. The first longitudinal clearance 1238 and/or a second longitudinal clearance 1239, also referred to as gaps, are positioned between the proximal surfaces of clevis 1223 and the opposing distal surfaces of the connection link 1241. In some embodiments, the first longitudinal clearance 1238 and/or a second longitudinal clearance 1239 can be at least partially filled or completely filled with a compressible material, such as elastomer, polymer, rubber, foam, sponge material, or combinations of these. In other embodiments, a compressible element, such as compressible element 1464 of FIGs. 14A and 14B, can be positioned at the first longitudinal clearance 1238 and/or a second longitudinal clearance 1239, such as a spring, a compressible disk such as an elastomeric disk, a hydraulic piston, a pneumatic piston, or combinations thereof. The introduction of a material or device 1464 at the gaps 1238, 1239 can provide for additional stability, and can absorb shock or force-related event that may occur during operation.

The longitudinal clearances 1238, 1239 are dimensioned in a longitudinal direction to prevent or minimize contact between the clevis 1223 and a distal link 1241 of the first assembly 1221 when a force is imparted by the movement of the activation element 1420. The dimension of the longitudinal clearances 1238, 1239 can include a length, width, area, or other well-known dimension. The dimension of the longitudinal clearances 1238, 1239 also provide for play, or "wiggle room", between the clevis 1223 and the distal link 1241 of the first assembly 1221 when a force is imparted by a movement of the activation element 1420, for example, in a longitudinal direction relative to the direction of extension of the first assembly 1221. Therefore, the dimension of at least one of the first longitudinal clearances 1238 and the second longitudinal clearance 1239, for example, a gap width, can be reduced when a force is imparted, preventing the imparted force from imparting another force on the first assembly 1221, in particular, the steering cables 1410 and/or links 1236 at the articulation region 1235. Accordingly, the first longitudinal clearance 1239 and/or the second longitudinal clearance 1238 can ensure the isolation of the imparted force from the first assembly 1221, and prevent binding or locking of the first assembly 1221.

The first longitudinal clearance 1238 can be between the base 1213 of the clevis 1223 and the distal end 1217 of the first assembly 1221. The second longitudinal clearance 1239 can be between an outermost end of the protrusion 1214 of the clevis 1223 and an inner end wall 1216 of the recess at the distal link 1241 of the first assembly 1221.

In an embodiment, the articulation region 1235 of the first assembly 1221 includes a plurality of articulation segments 1236, or links. The articulation segments 1236 can be the same as or similar to the segment links 236 described above with respect to FIGs. 2-9. Details of the articulation segments 1236 are therefore not repeated for brevity.

At least two steering cables 1410 can extend through the tool shaft 1220 to the articulation region 1235 for controlling an articulation of the articulation segments 1236. Each of the steering cables 1410 has a proximal end that can terminate at the surgical tool handle 1205. Movement of the articulation segments 1236 relative to each other can be controlled by the handle 1205.

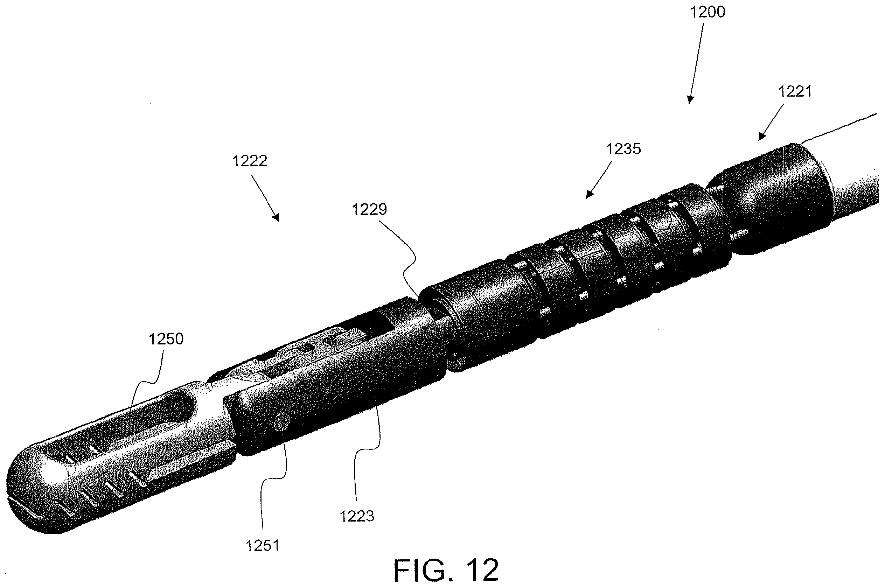

**FIG. 12** is an oblique view of the articulating surgical tool 1200 of FIGs 10 and 11, in accordance with another embodiment of the present inventive concepts. As shown in FIG. 12, the clevis 1223 can have a cylindrical outer surface that includes at least one first flat portion 1229 that registers with a corresponding second flat portion (not shown) of the inner surface of the distal end. The registration of the first flat portion 1229 and the second flat portion prevents twisting of the second assembly 1222 relative to the first assembly 1221.

The functional mechanism 1250 can include a grasper that is coupled to the clevis 1223 at two pivot points. Although a grasper 1250 is shown, other functional mechanisms can equally apply. Here, a first pin 1251 can extend through a hole at a first side of the clevis 1223, and a second pin (not shown) can extend through a hole at a second side of the clevis 1223 opposite the first side. As shown in FIG. 12, the grasper 1250 can extend along a same direction of extension as an articulation region 1235 and a first assembly 1221 of the surgical tool 1200. The pivot regions permit the grasper 1250 to move in a direction that is tangential to the direction of extension during an operation, for example, by controlling one or more articulation cables (not shown) from a handle, joystick, or other controller device.

**FIG. 13** is an expanded view of the handle assembly 1205 illustrated in FIG. 10, in accordance with another embodiment of the present inventive concepts. The handle assembly 1205 can be coupled to a proximal end of the support element 1231 at a proximal end of the first assembly 1221. At least one steering cable 1410 can be coupled to the handle 1205 and/or the ball and socket assembly 1212 to control articulation of the first assembly 1221. A locking mechanism 1462 such as a threaded nut, thumb screw, or similar device can extend through the ball and socket assembly, for locking a position of the ball and socket assembly 1212, which can hold the steering cables 1410 in place.

The activation element 1420 can be coupled to the trigger 1208. The trigger 1208 can be spring-loaded, for example, including a spring assembly 1251 between the trigger 1208 and the handle 1205. The trigger 1208 when pulled or otherwise activated can induce a motion of the activation element 1420. In particular, the activation element 1420 can move in a direction towards the handle assembly 1205. A release of the trigger 1208 by an operator causes the trigger 1208 to reset in turn allowing the activation element 1420 to move in an opposite direction, for example, toward the functional mechanism 1250.

The handle assembly 1205 includes one or more mounts 1246 which secure a proximal end of the support element 1231 to the handle 1205. The mount 1246 can include two or more compressible elements that hold the support element 1231 in place between them. The activation element 1420 can extend through the mount 1246 to the trigger 1208.

**FIGs. 14A and 14B** illustrate an operation of an articulating surgical tool 1400, in accordance with an embodiment. In particular, **FIG. 14A** is cross-sectional side view of an articulating surgical tool 1400 having a functional element in an open state, and **FIG. 14B** is cross-sectional side view of the articulating surgical tool 1400 of FIG. 14A, where the functional element is in a closed state. The surgical tool 1400 is similar to the surgical tool 1200described herein with reference to FIGs. 10-13, with one notable exception: a different functional mechanism 1450 is attached to a clevis 1423, for example, a pair of surgical scissors.

The scissors 1450 can include two blades 1455, 1456 attached to each other at a fulcrum 1452. A spring-biased element 1451 can be attached between the blades 1455, 1456 to maintain the scissors 1450 at an open position as shown at FIG. 14A. An arm 1457, 1458 can be movably coupled to each blade 1455, 1456, respectively, at a pivot point 1459. An attachment 1453 can be movably attached to an outermost end of each arm 1457, 1458 at a pivot point 1459. The four pivot points 1459 permit the scissors 1450 to open and close when a force is applied to the attachment 1453.

A distal end of an activation element 1420 is coupled to the scissors attachment 1453. A proximal end of the activation element 1420 is coupled to the handle trigger 1408 of a handle 1405.

In the first state, or open state, shown in FIG. 14A, the functional mechanism 1450 is in an equilibrium condition, where no force is applied from the attachment 1453 to the scissors 1450.

The spring-biased element 1452 can operate to apply a force relative to the movement of the activation element 1420. In the first state, the spring-biased element 1452 applies a force between the blades 1455, 1456 and can reset the functional mechanism 1450. In the first state, no force is applied by the steering cables 1410a, 1410b, i.e., Fsc1=0, Fsc2=0. Also, no force is applied by the activation element 1420, i.e., Fa1=0.

In the second state shown in FIG. 14B, a force is imparted by the activation element 1420, i.e., Fa1>0. The force Fa1 can be imparted by squeezing (S) the trigger 1408, or other device that pulls the activation element 1420 in a direction away from the scissors 1450. In doing so, the activation element 1420 can move in a linear direction within the lumen of the support element 1431, which is held in place in the surgical tool 1400. The force Fa1 imparted by the movement of the activation element 1420 is isolated from the first assembly 1421 by the support element 1431. Accordingly, the force Fa1 imparted by the movement of the activation element 1420 does not affect or change the forces applied by the steering cables 1410a, 1410b, e.g., Fsc1=0, Fsc2=0. In another example, forces applied by the steering cable, e.g., Fsc1=x, Fsc2=y, during movement of the activation element 1420 are not changed by the force Fa1 imparted by the movement of the activation element 1420.
In this manner, the isolation of the force imparted by the movement of the activation element 1420 relative to the first assembly 1421 by the support element 1431 prevents binding at an articulation region 1435 of the first assembly 1421. For example, the steering cables 1410 can be prevented from binding or locking up as a result of movement of the activation element 1420. Such isolation of the forces as provided by the embodiments of the present inventive concepts is not necessarily perfect or complete, as a marginal amount of force transfer to elements of the first assembly 1421 can occur, while still preventing or largely mitigating any binding or locking at the articulation region 1435 of the first assembly 1421.

The longitudinal clearances 1438, 1439 can also contribute to preventing the force Fa1 from impacting the steering cable forces Fsc1, Fsc2. For example, the longitudinal clearance 1439 can be reduced from a width W to a width W' when the force Fa1 is imparted, preventing the imparted force Fa1 from imparting the other forces, namely, Fsc1, Fsc2.

In the illustrated embodiment, a compressible element 1464 can be positioned at the first longitudinal clearance 1238 and/or a second longitudinal clearance 1239, such as a spring, a compressible disk such as an elastomeric disk, a hydraulic piston, a pneumatic piston, or combinations thereof. The introduction of a material or device 1464 at the gaps 1238, 1239 can provide for additional stability, and can absorb shock or force-related event that may occur during operation.

**FIGs. 15A-15C** are cross-sectional side views of an articulating surgical tool 1500, each side view representing a different state of the articulating surgical tool 1500, in accordance with another embodiment of the present inventive concepts. Here, the second assembly 1522 does not extend through the first assembly 1521. Instead, the second assembly 1522 is adjacent the first assembly 1521, for example, extending along a common direction of extension. The first assembly 1521 and the second assembly 1522 are co-located in a lumen of a sheath 1581. The second assembly 1522 includes a support element 1531 and an activation element 1520 that moves freely proximal to an outer surface of the support element 1531. Accordingly, a central opening, referred to above as a first cable channel, may not be required at the first assembly 1521, since an activation element 1520 does not extend through the first assembly 1521; rather, the activation element 1520 extends along an outer surface of the first assembly 1521. Here, the first assembly 1521 can include openings, referred to above as second cable channels, for receiving one or more steering cables 1510 which can control a movement of the articulation region 1535 of the first assembly 1521. Articulation region 1535 terminates at distal link 1580. Distal link 1580 may be an atraumatic tip, such as a tip designed to prevent trauma to tissue, such as during operation or insertion of surgical tool 1500.

A functional element 1550, for example, scissors, grasper, and so on, can be coupled to the activation element 1520, which controls the movement of the functional element 1550 by applying a force in a manner similar to that described herein. A mount (not shown) such as a clevis can be positioned between the functional element 1550 and a distal end of the second assembly 1522.

In FIG. 15A, the surgical tool 1500 is in a first state, where the first and second assemblies 1521, 1522 extend in a longitudinal direction of extension. The functional element 1550 can be in a state of equilibrium, i.e., the blades are open due to a spring-biased element (not shown) attached between the blades similar to that shown in FIGs. 14A and 14B, and further due to no tension being applied to the activation element 1520. Here, no force Fa1 of the activation element 1520 is applied, and no forces Fsc1, Fsc2 of the first and second steering cables 1510 are applied.

In FIG. 15B, the surgical tool 1500 is in a second state, where the first and second assemblies 1521, 1522 extend in a longitudinal direction of extension, and the functional element 1550 is closed due to a force Fa1 being at the activation element 1520. For example, the activation element 1520 is being pulled in a direction away from the functional element 1550. Here, the force Fa1 imparted by the movement of the activation element 1520 is isolated from the articulation region 1535 of the first assembly 1521, thereby preventing the links at the articulation region 1535 from being pressed against each other which can otherwise lock them up. Binding at the steering cables 1510 can also be prevented. No forces Fsc1, Fsc2 of the first and second steering cables 1510 are applied.

In FIG. 15C, the surgical tool 1500 is in a third state, where the first and second assemblies 1521, 1522 are bent or angled relative to a longitudinal direction of extension due to forces Fsc1 and/or Fsc2 being applied to the steering cables 1510, i.e., Fsc1<0, Fsc2>0. Also, the functional element 1550 is opened similar to FIG. 15A due to no force Fa1 at the activation element 1520, i.e., Fa1=0. Here, the forces Fsc1, Fsc2 applied by the steering cables 1510 do not affect the force Fa1 applied by the activation element 1520. Therefore, the risk of an unintentional opening or closing of the functional element 1550 is reduced or eliminated during movement of the articulation region 1535.

While the present inventive concepts have been particularly shown and described above with reference to exemplary embodiments thereof, it will be understood by those of ordinary skill in the art, that various changes in form and detail can be made without departing from the spirit and scope of the present inventive concepts described and defined by the following claims.

## Claims

1. A surgical tool, comprising:
an elongated first assembly (1221), comprising:
an articulation region; and
a distal end (1217) comprising a recess, the recess comprising an inner end wall (1216) and a sidewall;
an elongated second assembly (1222), comprising:
an elongated support element (1231);
a clevis (1223), comprising a base (1213) and a protrusion extending from the base (1213), the clevis (1223) coupled to the elongated support element (1231); and
an elongated activation element (1420) moveable relative to the support element (1231);
a functional mechanism (1250) coupled to the activation element (1420), a movement of the functional mechanism (1250) being in response to a movement of the activation element (1420), wherein a force imparted by the movement of the activation element (1420) is isolated from the first assembly (1221) by the support element (1231);
a first longitudinal clearance (1239) between the clevis (1223) and the distal end (1217) of the first assembly (1221);
a second longitudinal clearance (1238) between the protrusion (1214) of the clevis (1223) and the inner end wall (1216) of the recess.

2. The surgical tool of claim 1 wherein
a) the clevis is coupled to a distal end of the support element, and/or
b) an inner surface of the clevis is coupled to an outer surface of the support element, and/or
c) the clevis is bonded to the support element, in particular wherein
the bond includes an adhesive, and/or
the clevis is welded to the support element, and/or wherein
the clevis and the support element are coupled by at least one of swaging, threading, pinning, snap-fitting, press-fitting, or coupling together, and/or
d) the activation element moves freely along a direction of extension of the support element and the clevis.

3. The surgical tool according to at least one of the preceding claims, wherein the first longitudinal clearance is dimensioned, in a longitudinal direction, to prevent contact between the clevis and the distal end of the first assembly when the force is imparted by the movement of the activation element, in particular wherein,
a) the dimension of the first longitudinal clearance ensures the isolation of the imparted force, and/or
b) the dimension of the first longitudinal clearance provides for play between the clevis and the distal end of the first assembly when the force is imparted, and/or
the dimension of the first longitudinal clearance is reduced when the force is imparted.

4. The surgical tool according to at least one of the preceding claims, further comprising a compressible material positioned in the first longitudinal clearance, in particular
wherein the compressible material comprises at least one of elastomer, polymer, rubber, foam, sponge material, or combinations thereof.

5. The surgical tool according to at least one of the preceding claims, further comprising a compressible element positioned in the first longitudinal clearance, in particular,
wherein the compressible element comprises at least one of a spring, a compressible disk, an elastomeric disk, a hydraulic piston, a pneumatic piston, or combinations thereof.

6. The surgical tool according to at least one of the preceding claims, wherein
a) the base is wider than the protrusion, and/or
b) the first longitudinal clearance is between the base of the clevis and the distal end of the first assembly, and/or
c) an outer width of the base is equal to an outer width of the distal end of the first assembly, and/or
d) the protrusion has a cylindrical outer surface and the distal end has a cylindrical inner surface, in particular
wherein the cylindrical outer surface of the protrusion includes at least one first flat portion that registers with a corresponding second flat portion of the inner surface of the distal end, further in particular wherein the registration of the first and second flat portions prevents twisting of the second assembly relative to the first assembly, and/or
e) the clevis includes a housing for receiving the functional mechanism, in particular wherein
the activation element is coupled to the functional mechanism at the housing, further in particular wherein
the housing is dimensioned to permit the functional mechanism to expand and contract relative to the housing during the movement of the functional mechanism.

7. The surgical tool according to at least one of the preceding claims, wherein
a) the second assembly is in communication with the first assembly so that the support element of the second assembly is movable relative to the first assembly, and/or
b) the support element includes a lumen that extends along a direction of extension of the support element, in particular wherein,
the activation element is slidably positioned in the lumen of the support element, and/or
c) the activation element slidably communicates with the support element in a direction of extension of the support element.

8. The surgical tool according to at least one of the preceding claims wherein
a) the support element is constructed and arranged as a coil, and/or
b) the support element is constructed and arranged as a rod, in particular wherein,
the rod has limited compression in a direction of extension of the support element and is flexible in a lateral direction relative to the direction of extension, and/or
c) the support element is constructed and arranged as a hollow tube, and/or
d) the support element comprises an arrangement of multiple links.

9. The surgical tool according to at least one of the preceding claims wherein
a) the support element has limited compression in a direction of extension of the support element and is flexible in a lateral direction relative to the direction of extension, and/or
b) the support element absorbs a load caused by the force imparted by the movement of the activation element, and/or
c) the support element prevents a force from being applied to the first assembly when the force is imparted by the movement of the activation element.

10. The surgical tool according to at least one of the preceding claims wherein
a) the activation element is freely moveable relative to the support element, and/or
b) the activation element moves freely within the support element, and/or
c) the activation element moves freely proximal to an outer surface of the support element, and/or
d) movement of the activation element is induced by a handle at a proximal end of the surgical tool, and/or
e) the activation element is constructed and arranged as a wire, in particular wherein,
the wire is constructed and arranged to deliver energy and/or data, and/or
f) the activation element is constructed and arranged as a cable, and/or
g) activation element is constructed and arranged as a fiber, in particular wherein
the fiber is constructed and arranged to deliver energy and/or data, and/or
the activation element comprises a lubricious outer surface portion, in particular wherein
the lubricious outer surface portion comprises a material selected from the group consisting of: Teflon; graphite; a hydrophilic coating; a surface area reducing texture; and combinations thereof.

11. The surgical tool according to at least one of the preceding claims wherein
a) the functional mechanism comprises at least one of: a grasper; a scissor; a cutter; a claw; or a knife, and/or
b) the functional mechanism comprises at least one of: an ablator, a cauterizer, a drug delivery apparatus, a radiation source, an EKG electrode, a pressure sensor, a blood sensor, a camera, a magnet, a heating element or a cryogenic element, and/or
c) functional mechanism comprises a spring-biased tool, in particular wherein,
the spring-biased tool operates to apply a force relative to the movement of the activation element, and/or
the spring-biased tool operates to apply a force to reset the functional mechanism, and/or
the spring-biased tool operates to apply a force that is opposite the force imparted by the movement of the activation element,
d) the functional element is constructed and arranged to articulate with respect to a direction of extension of the first assembly, and/or
e) the functional mechanism includes an actuating piston coupled to the activation element to link the activation element to the functional mechanism, in particular wherein
the actuating piston is positioned within an inner cavity of a distal end of the first assembly, further in particular
wherein the functional element further includes first and second actuation link members coupled to the actuating piston, further in particular,
wherein the first and second actuation link members include a material selected from the group consisting of: metal, plastic, a thermoplastic polymer, stainless steel, polyvinyl chloride; liquid-crystal polymer; and combinations thereof, and/or
the functional element further includes first and second claw members respectively coupled to the first and second actuation link members, in particular wherein,
the first and second claw members include a material selected from the group consisting of: metal, plastic, a thermoplastic polymer, stainless steel, polyvinyl chloride; liquid-crystal polymer; and combinations thereof, and/or
linear movement of the actuating piston within the inner cavity causes the first and second claw members to open and close.

12. The surgical tool according to at least one of the preceding claims further comprising a handle coupled to a proximal end of the support element at a proximal end of the first assembly, wherein
a) the handle controls the surgical tool, and/or
b) further comprising at least one steering cable that operates to control articulation of the first assembly, wherein the at least one steering cable is coupled to the handle, in particular wherein
further comprising a ball and socket mechanism in communication with the handle that controls movement of the at least one steering cable, further in particular
further comprising a locking mechanism that locks a position of the ball and socket mechanism, further in particular
wherein the locking mechanism comprises a threaded nut or a thumb screw, and/or
c) the activation element is coupled to the handle, in particular wherein
the handle includes a trigger and wherein the activation element is coupled to the trigger, further in particular,
wherein
aa) the trigger is spring-loaded, further in particular,
wherein activation of the trigger moves the activation element in a first direction, further in particular
wherein release of the trigger by an operator causes the trigger to reset in turn allowing the activation element to move in a second direction opposite the first direction, and/or
bb) the trigger initiates the movement of the activation element in a direction toward the trigger, and/or
d) the handle includes a mount at which the support element is coupled, in particular wherein
the support element is coupled to the mount at a proximal end of the support element, and/or
the handle further includes a trigger and wherein the activation element extends through the mount to the trigger, and/or
e) the handle includes one selected from the group consisting of: scissor handles; a palm-held grip; a thumb/index/middle finger grip; a pistol grip; a reciprocating trigger; and combinations thereof.

13. The surgical tool according to at least one of the preceding claims, wherein
a) the surgical tool includes a locking device constructed and arranged to lock an articulated position of the functional mechanism, and/or
b) the surgical tool includes a locking device constructed and arranged to lock an operational mode of the functional mechanism.

14. The surgical tool according to at least one of the preceding claims, wherein
a) the first assembly is adjacent an outer surface of the second assembly, and/or
b) the first and second assemblies extend in a same direction of extension, and/or
c) the first assembly and the second assembly are co-located in a lumen of an articulating probe, and/or
d) at least a portion of the second assembly is positioned in the first assembly, and/or
e) the isolation of the force imparted by the movement of the activation element relative to the first assembly by the support element prevents binding at an articulation region of the first assembly, and/or
f) the isolation of the force imparted by the movement of the activation element relative to the first assembly by the support element prevents binding of articulation segments at an articulation region of the first assembly.

15. The surgical tool according to at least one of the preceding claims, wherein the surgical tool is constructed and arranged to be controlled via a human interface device, in particular wherein,
the human interface device includes one selected from the group consisting of: a haptic controller; a joystick; a track ball; a mouse; an electromechanical device; and combinations thereof.

16. The surgical tool according to at least one of the preceding claims wherein the first assembly comprises:
a tool shaft;
an articulation region comprising a plurality of articulation segments; and
at least two steering cables extending through the tool shaft to the articulation region.

17. The surgical tool of claim 17 wherein
a) the articulation region is at a distal end of the surgical tool, and/or
b) the at least two steering cables control an articulation of the articulation segments, in particular wherein
aa) at least of the articulation segments includes at least one articulation cable channel through which a steering cable of the at least two steering cables extends, further in particular
wherein the at least one articulation cable channel comprises first through third articulation cable channels that are spaced approximately 120° apart around a circumference or perimeter of the articulation segment, and/or
the at least one articulation cable channel comprises first through fourth articulation cable channels that are spaced approximately 90° apart around a circumference or perimeter of the articulation segment, and/or
the at least one articulation cable channel comprises first through fourth articulation cable channels that are spaced approximately 90° apart from one another along a common radial path relative to a center axis of the first assembly, and/or
bb) the at least two steering cables operate to lock an articulation position of the articulation region, in particular wherein
further comprising a locking mechanism that retains the steering cables in a locked position, and/or
c) the force imparted by the movement of the activation element is isolated from the articulation region by the support element, in particular
wherein
the force imparted by the movement of the activation element is independent from an orientation of the articulation region, and/or
the force imparted by the movement of the activation element is independent from a force applied to the at least two steering cables, and/or
the isolation of the force imparted by the movement of the activation element from the articulation region prevents binding of the at least two steering cables, and/or
the isolation of the force imparted by the movement of the activation element from the articulation region prevents inadvertent locking of neighboring articulation segments, and/or the isolation of the force imparted by the movement of the activation element from the articulation region avoids movement of the articulation region in response to a movement of the activation element, and/or
the tool shaft includes a lumen guiding member for receiving the activation element and the at least two steering cables, in particular wherein,
the lumen guiding member includes a multi-lumen stiffening rod, further in particular wherein
the multi-lumen stiffening rod includes a first cable channel for receiving the activation element and a plurality of second cable channels for receiving the at least two steering cables, and/or
d) the articulation region of the tool shaft includes at least two segment links, in particular wherein,
d1) a first segment link of the at least two segment links is coupled to a first shaft portion of the tool shaft, and a second segment link of the at least two segment links is in communication with the functional mechanism, further in particular, wherein
aa) the articulation region of the tool shaft further includes one or more third segment links coupled between the first segment link and the second segment link, and/or
bb) the first segment link includes a body having a first portion and a second portion, wherein the second portion includes a semi-spherical body portion, and/or
cc) the first segment link includes a body having a first portion and a second portion, wherein the second portion includes a convex body portion, in particular wherein
the convex body portion is a semi-spherical body portion, and/or
the convex body portion is a semi-ellipsoidal body portion, and/or
the first portion includes a cylindrical body portion, and/or
dd) a semi-spherical body portion of the first segment link mates with a semi-spherical cavity portion of the first shaft portion, and/or
ee) a semi-spherical body portion of the first segment link mates with a concave cavity portion of the first shaft portion, and/or
ff) the second segment link includes a body having a first portion and a second portion, wherein the second portion includes a convex body portion, in particular wherein,
the convex body portion is a semi-spherical body portion, and/or
the convex body portion is a semi-ellipsoidal body portion, and/or
the first portion includes a cylindrical body portion, and/or
d2) a bottom surface of a first portion of a first segment link of the plurality of segment links abuts an upper surface of a first portion of a second segment link of the plurality of segment links to restrict an angle of articulation with respect to a center axis of each of the first and second segment links, in particular wherein
the angle of articulation is restricted to approximately 12° to 15°, and/or
d3) each segment link of the at least two is constructed and arranged to provide approximately 12° to 15° of articulation between the functional mechanism and a working surface of the surgical tool, and/or
d4) each segment link of the at least two is constructed and arranged to provide approximately 12° to 15° of articulation between the functional mechanism and a working surface of the tool shaft, and/or
d5) the articulation region is constructed and arranged to support a force of 1 lb_{F} without deflecting more than approximately ½ inch, and/or
d6) the articulation region is constructed and arranged to support a force of approximately 1 lb_{F} without deflecting more than approximately ½ inch when in a fully articulated state, and/or
d7) a distal end of the support element is positioned at the second segment link, and/or
e) the articulation region of the first assembly includes a lumen and wherein a portion of the second assembly is positioned in the lumen of the first assembly, or
f) each of the at least two steering cables has a proximal end terminating at a surgical tool handle, and wherein the movement of the articulation segments relative to each other is controlled by the surgical tool handle.

18. The surgical tool according to at least one of the preceding claims wherein the first assembly is adjacent an outer surface of the second assembly and extends along a common direction of extension as the second assembly, and/or
further comprising a sheath, the first assembly and the second assembly co-located in a lumen of a sheath, and/or
the activation element extends along an outer surface of the first assembly.
